# EUROPEAN PATENT SPECIFICATION

(11) **EP 1 858 857 B1**
(45) Date of publication and mention of the grant of the patent: **08.12.2010**
(21) Application number: 06721069.0
(22) Date of filing: 27.02.2006
(51) Int. Cl.: C07D 221/22, C07D 413/10, C07D 401/10, C07D 417/10, C07D 405/10, A61K 31/439, A61P 25/00, A61P 9/00

(54) **AZABICYCLOALKANE DERIVATIVES USEFUL AS NICOTINIC ACETYLCHOLINE RECEPTOR AGONISTS**
ALS AGONISTEN FÜR DEN NIKOTINISCHEN ACETYLCHOLINREZEPTOR GEEIGNETE AZABICYCLOALKANDERIVATE
DERIVES D' AZABICYCLOALCANE UTILES EN TANT QU'AGONISTES DU RECEPTEUR NICOTINIQUE DE L'ACETYLCHOLINE

(30) Priority: 04.03.2005 US 658444 P
(43) Date of publication of application: 28.11.2007
(73) Proprietor: Eli Lilly & Company, Indianapolis, IN 46285 (US)
(72) Inventor: MARTIN, Fionna Mitchell, Eli Lilly and Company Ltd, Basingstoke Hampshire RG21 6XA (GB); FLYNN, Claire June, Eli Lilly and Company Ltd, Basingstoke Hampshire RG21 6XA (GB); RICHARDS, Simon James, Eli Lilly and Company Ltd, Basingstoke Hampshire RG21 6XA (GB)
(74) Representative: Ingham, Stephen H.
(86) International application number: PCT/US2006/006756
(87) International publication number: WO 2006/096358

(56) References cited:
- WO-A-01/44243
- WO-A-03/004493

## Description

### Field of the invention

The present invention relates to compounds that modulate neurotransmission by promoting the release of neurotransmitters such as acetylcholine, dopamine and norepinephrine. More particularly, the invention relates to compounds or pharmaceutically acceptable salts thereof, processes for their preparation, pharmaceutical compositions which contain them and their uses in therapy, wherein said compounds are able to bind to and modulate nicotinic acetylcholine receptors in a mammal.

### Background to the invention

Acetylcholine receptors modulate the release of neurotransmitters such as for example dopamine, norepinephrine, acetylcholine, and serotonin from different brain regions. By such action, acetylcholine receptors participate in the modulation of neuroendocrine function, respiration, mood, motor control and function, focus and attention, concentration, memory and cognition, and the mechanisms of substance abuse.

Ligands for acetylcholine receptors have been demonstrated to have effects on attention, cognition, appetite, substance abuse, memory, extrapyramidal function, cardiovascular function, pain, and gastrointestinal motility and function. The distribution of acetylcholine receptors that bind nicotine, i.e., nicotinic acetylcholine receptors, is widespread in the brain, including being found in the basal ganglia, limbic system, cerebral cortex and mid- and hind-brain nuclei. In the periphery, their distribution includes being in muscle, autonomic ganglia, the gastrointestinal tract and the cardiovascular system.

Nicotinic acetylcholine receptors (nAChRs) belong to the ligand gated ion channel family of neurotransmitter receptors. In neuronal and peripheral tissue, nAChRs possess a pentameric structure consisting of 5 protein subunits surrounding a central ion channel. Five neuromuscular subunits (α, β, γ, δ, ε), ten peripheral or neuronal α-subunits (α1 to α10), and three peripheral or neuronal β-subunits (β2 to β4) have been identified. These subunits combine to form pentameric receptors in three ways: first, with homomeric 5[α] stoichiometry, for example, α7 to α9; second, with heteromeric 2[α]3[β] stoichiometry, for example, combinations of α1 to α6 and β2 to β4 subunits; and third, the 2[α]1[β]1[δ]1[γ/ε] stoichiometry found in neuromuscular receptors.

Nicotine modulates multiple neuronal, peripheral and neuromuscular subtypes of nAChRs. While demonstrating beneficial effects in a number of neuronal diseases mediated by nAChRs, nicotine also demonstrates a number of undesirable side effects on cardiovascular, gastrointestinal and neuromuscular systems. It will be appreciated that there is a need for compounds that can selectively modulate a single or specific group of nAChRs.

WO 2005/007655 describes heteroaryl fused azapolycyclic compounds that bind to neuronal nicotinic acetylcholine specific receptors sites. These compounds are said to be useful in modulating cholinergic function and thus suitable for reducing nicotine addiction and treating a series of disorders associated with cholinergic function.

Azabicylic compounds as central nervous system active agents useful for treating pain are disclosed by WO 2004/016604. These include compounds of the formula: 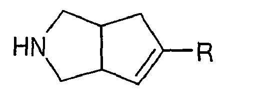 wherein R is an aryl or heterocycle.

WO 01/44243 describes diazabicyloalkanes and in particular heteroaryl-diazabicyclo [3.3.1]nonane derivatives of the general formula: 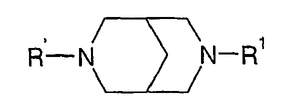 wherein R' represents hydrogen, an alkyl group, an aryl group, an aralkyl group or a fluorescent group; and R¹ represents an optionally substituted mono- or poly-heterocyclic group. Similar heteroaryl-diazabicyclo[3.3.1]nonane compounds are disclosed by WO 02/096911 as cholinergic ligands.

WO03/004493 discloses a generic formula relating to compounds with an aromatic ring system covalently bonded to an azabicylic group of formula: 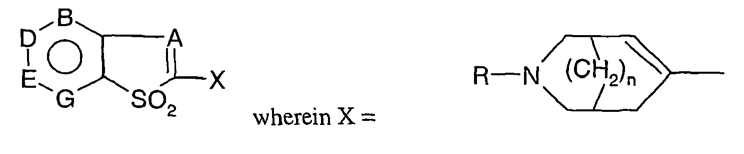 wherein R represents hydrogen, alkyl, alkenyl, alkynyl, cycloalkyl, cycloalkylalkyl, aryl and aralkyl.

There is a need to provide novel compounds which can act as agonists of nAChRs, showing a high binding affinity thereto. Furthermore, there is a need to provide agonists of nAChRs which can selectively modulate a single or specific group of nAChRs and in so doing extend the range of compounds available for the treatment of mammalian disorders associated therewith, while minimizing the potential for such compounds to exert adverse side effects. In particular, the applicant identifies a need to provide new selective agonists of α4β2 nicotinic acetylcholine receptors.

The objective technical problem addressed by the present invention therefore relates to the provision of novel agents that may bind to and modulate α4β2 nicotinic acetylcholine receptors.

### Summary of the invention

In a first aspect the present invention provides a compound of formula I or a pharmaceutically acceptable salt thereof: 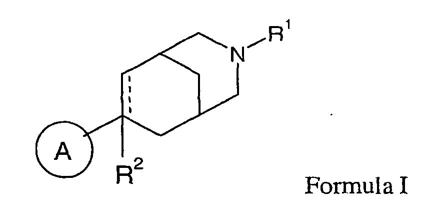 wherein,
- R¹: is hydrogen, C₁₋₆alkoxycarbonyl, C₁₋₆alkyl, benzyloxycarbonyl, cyanoC₁₋₆alkyl, dihydro-3-pyridinylcarbonyl, hydroxy, hydroxyC₁₋₆alkyl, phenoxycarbonyl, -NR³R⁴, (NR³R⁴)C₁₋₄alkyl and (NR³R⁴)carbonylC₁₋₄alkyl;
- R²: is present as H or OH;
- -----: is an optional double bond, wherein ----- when is a double bond R² is absent;
- A: is an aryl selected from phenyl, naphthyl, indenyl, indanyl, azulenyl and tetrahydronapthyl,
wherein said aryl is optionally substituted with one, two, three or four substituents independently selected from C₂₋₆alkenyl, C₁₋₆ alkoxy, C₁₋₆alkoxyC₁₋₄alkoxy, C₁₋₆alkoxyC₁₋₄alkyl, C₁₋₆alkoxycarbonyl, C₁₋₆alkoxycarbonylC₁₋₄alkyl, C₁₋₆alkyl, C₁₋₆alkylcarbonyl, C₁₋₆alkylcarbonyloxy, C₁₋₆alkylthio, C₂₋₆alkynyl, carboxy, carboxylC₁₋₆alkyl, cyano, cyanoC₁₋₆alkyl, C₃₋₆cycloalkyl, C₃₋₆cycloalkylC₁₋₄alkyl, haloC₃₋₆cycloalkylC₁₋₄alkyl, C₃₋₆cycloalkylC₁₋₄alkoxy, haloC₃₋₆cycloalkylC₁₋₄alkoxy, formyl, formylC₁₋₆alkyl, haloC₁₋₆alkoxy, haloC₁₋₆alkyl, halogen, hydroxy, hydroxyC₁₋₆alkyl, mercapto, mercaptoC₁₋₆alkyl, nitro, triphenylmethyl (trityl), a heteroaryl optionally substituted by one, two or three methyl substituents, wherein said heteroaryl is selected from furyl, thienyl, pyrrolyl, pyrazolyl, imidazolyl, triazolyl, oxazolyl, isoxazolyl, thiazolyl, thiadiazolyl, isothiazolyl, oxadiazolyl, oxatriazolyl, pyridinyl, pyridazinyl, pyrimidinyl, pyrazinyl, triazinyl and tetrazolyl, -C(NH)NR³R⁴, -NR³R⁴, -C₁₋₄alkyl (NR³R⁴), -CO(NR³R⁴), -C₁₋₄alkylCO(NR³R⁴), -S(O)₂ NR³R⁴, - NR⁵S(O)₂ R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂ R⁵, phenyl and phenyl(CH₂)ₙO, wherein said phenyl or phenyl(CH₂)ₙO is optionally substituted with one, two or three substituents independently selected from chlorine, fluorine, cyano, C₁₋₃alkyl and C₁₋₃alkoxy, wherein said C₁₋₃alkyl or éC₁₋₃alkoxy is optionally substituted with 1 to 3 fluorines; or where two substituents on adjacent carbon atoms of said aryl together optionally represent - OCH₂CH₂-, -OCH₂CH(OH)-, -OCH₂CH₂O- or -OCH₂O-;
R³ and R⁴ are independently selected from hydrogen, C₁₋₄alkyl and C₁₋₄alkylcarbonyl; R⁵, R⁶ and R⁷ are independently selected from hydrogen, C₁₋₄alkyl, phenyl and phenylC₁₋₄alkyl;
n is 0, 1 or 2.

The invention also relates to pharmaceutically acceptable compositions which contain a compound according to the invention and therefore in a second aspect the invention provides a pharmaceutical composition comprising a compound according to formula I.

In a third aspect the invention relates to a compound according to the invention for use in therapy.

In a fourth aspect the invention relates to the use of an effective amount of a compound according to the invention in the preparation of a medicament for the treatment or prevention of one or more conditions in a mammal selected from inflammatory bowel disease, ulcerative colitis, pyoderma gangrenosum,

Crohn's disease, irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotrophic lateral sclerosis (ALS), cognitive dysfunction, hypertension, bulimia, anorexia, obesity, cardiac arrhythmias, gastric acid hypersecretion, ulcers, pheochromocytoma, progressive supranuclear palsy, chemical dependencies and addictions, dependencies on, or addictions to nicotine (or tobacco products), alcohol, benzodiazopines, barbiturates, opioids or cocaine, headache, migraine, stroke, traumatic brain injury (TBI), obsessive-compulsive disorder (OCD), psychosis, Huntington's chorea, tardive dyskinesia, hyperkinesia, dyslexia, schizophrenia, multi-infarct dementia, age-related cognitive decline, epilepsy, petit mal absence epilepsy, senile dementia of the Alzheimer's type (AD), Parkinson's disease (PD), attention deficit hyperactivity disorder (ADHD), attention deficit disorder (ADD), restless legs syndrome (RLS), mild cognitive impairment, cognitive enhancement in schizophrenia, drug induced extrapyramidal symptoms, conduct disorder, oppositional defiant disorder, anxiety in anxious smokers, cardiovascular risk in pregnancy, delayed ejaculation, emesis, diarrhoea, nicotine gum addiction, sleep prevention, ischemia, and Tourette's Syndrome.

In a fifth aspect the invention provides an intermediate to the formation of compounds according to formula I, wherein said intermediate has a structure according to formula II: 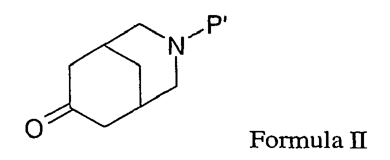 wherein P' is a nitrogen protecting group selected from benzyl, trityl, acyl or carbamates of t-butyl, benzyl, 2,4-dichlorobenzyl, 2-(biphenylyl)isopropyl, 9-fluorenylmethyl, isonicotinyl or allyl.

### Detailed description of the invention

The invention relates novel compounds of formula I or a pharmaceutically acceptable salt thereof: 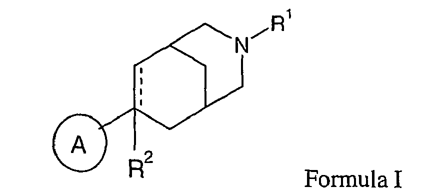 wherein,
- R¹: is hydrogen, C₁₋₆alkoxycarbonyl, C1-₆alkyl, benzyloxycarbonyl, cyanoC₁₋₆alkyl, dihydro-3-pyridinylcarbonyl, hydroxy, hydroxyC₁₋₆alkyl, phenoxycarbonyl, -NR³R⁴, (NR³R⁴)C₁₋₄alkyl and (NR³R⁴)carbonylC₁₋₄alkyl;
- R²: is present as H or OH;
- -----: is an optional double bond, wherein when ----- is a double bond R² is absent;
- A: is an aryl selected from phenyl, naphthyl, indenyl, indanyl, azulenyl and tetrahydronapthyl,
wherein said aryl is optionally substituted with one, two, three or four substituents independently selected from C₂₋₆alkenyl, C₁₋₆ alkoxy, C₁₋₆alkoxyC₁₋₄alkoxy, C₁₋₆alkoxyC₁₋₄alkyl, C₁₋₆ alkoxycarbonyl, C₁₋₆alkoxycarbonylC₁₋₄alkyl, C₁₋₆alkyl, C₁₋₆alkylcarbonyl, C₁₋₆alkylcarbonyloxy, C₁₋₆alkylthio, C₂₋₆alkynyl, carboxy, carboxyC₁₋₆alkyl, cyano, cyanoC₁₋₆alkyl, C₃₋₆cycloalkyl, C₃₋₆cycloalkylC₁₋₄alkyl, haloC₃₋₆cycloalkylC₁₋₄alkyl, C₃-₆cycloalkylC₁₋₄alkoxy, haloC₃₋₆cycloalkylC₁₋₄alkoxy, formyl, formylC₁₋₆alkyl, haloC₁₋₆alkoxy, haloC₁₋₆alkyl, halogen, hydroxy, hydroxyC₁₋₆alkyl, mercapto, mercaptoC₁₋₆alkyl, nitro, triphenylmethyl (trityl), a heteroaryl optionally substituted by one, two or three methyl substituents, wherein said heteroaryl is selected from furyl, thienyl, pyrrolyl, pyrazolyl, imidazolyl, triazolyl, oxazolyl, isoxazolyl, thiazolyl, thiadiazolyl, isothiazolyl, oxadiazolyl, oxatriazolyl, pyridinyl, pyridazinyl, pyrimidinyl, pyrazinyl, triazinyl and tetrazolyl, -C(NH)NR³R⁴, -NR³R⁴, -C₁₋₄alkyl (NR³R⁴), -CO(NR³R⁴), -C₁₋₄alkylCO(NR³R⁴), -S(O)₂ NR³R⁴, -NR⁵S(O)₂ R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂ R⁵, phenyl and phenyl(CH₂)ₙO, wherein said phenyl or phenyl(CH₂)ₙO is optionally substituted with one, two or three substituents independently selected from chlorine, fluorine, cyano, C₁₋₃alkyl and C₁₋₃alkoxy, wherein said C₁₋₃alkyl or C₁₋₃alkoxy is optionally substituted with 1 to 3 fluorines; or where two substituents on adjacent carbon atoms of said aryl together optionally represent -OCH₂CH₂-, -OCH₂CH(OH)-, -OCH₂CH₂O or -OCH₂O-;
R³ and R⁴ are independently selected from hydrogen, C₁₋₄alkyl and C₁₋₄alkylcarbonyl; R⁵, R⁶ and R⁷ are independently selected from hydrogen, C₁₋₄alkyl, phenyl and phenylC₁₋₄alkyl;
n is 0,1 or 2.

### Definition of terms:

Unless otherwise specifically defined, the term "aryl" as used herein, means a monocyclic ring system, or a fused bicyclic ring system wherein one or more of the fused rings are aromatic. Representative examples of aryl include, but are not limited to, azulenyl, indanyl, indenyl, naphthyl, phenyl and tetrahydronaphthyl.

The term "C₂₋₆alkenyl" as used herein, means a straight or branched chain hydrocarbon containing from 2 to 6 carbons, preferably in a straight chain, and containing at least one carbon-carbon double bond formed by the removal of two hydrogens. Representative examples of C₂₋₆alkenyl include, but are not limited to ethenyl, 2-propenyl, 2-methyl-2-propenyl, 3-butenyl, 4-pentenyl and 5-hexenyl.

The term "C₁₋₆alkoxy" as used herein, means a C₁₋₆alkyl group, as defined herein, appended to the parent molecular moiety through an oxy moiety, as defined herein. Representative examples of C₁₋₆alkoxy include, but are not limited to, methoxy, ethoxy, propoxy, 2-propoxy, butoxy, tert-butoxy, pentyloxy and hexyloxy.

The term "C₁₋₆alkoxyC₁₋₄alkoxy" as used herein, means a C₁₋₆alkoxy group, as defined herein, appended to the parent molecular moiety through another C₁₋₄alkoxy group, as defined herein. Representative examples of C₁₋₆alkoxyC₁₋₄alkoxy include, but are not limited to, tert-butoxymethoxy, 2-ethoxyethoxy, 2-methoxyethoxy and methoxymethoxy.

The term "C₁₋₆alkoxyC₁₋₄alkyl" as used herein, means a C₁₋₆alkoxy group, as defined herein, appended to the parent molecular moiety through a C₁₋₄alkyl group, as defined herein. Representative examples of C₁₋₆alkoxyC₁₋₄alkyl include, but are not limited to, tert-butoxymethyl, 2-ethoxyethyl, 2-methoxyethyl and methoxymethyl.

The term "C₁₋₆alkoxycarbonyl" as used herein, means a C₁₋₆alkoxy group, as defined herein, appended to the parent molecular moiety through a carbonyl group, as defined herein. Representative examples of C₁₋₆alkoxycarbonyl include, but are not limited to, methoxycarbonyl, ethoxycarbonyl and tert-butoxycarbonyl.

The term "C₁₋₆alkoxycarbonylC₁₋₄alkyl" as used herein, means a C₁₋₆alkoxycarbonyl group, as defined herein, appended to the parent molecular moiety through a C₁-₄alkyl group, as defined herein. Representative examples of C₁₋₆alkoxycarbonylC₁₋₄alkyl include, but are not limited to, 3-methoxycarbonylpropyl, 4-ethoxycarbonylbutyl, 2-(sec-butylcarbonyl)ethyl, 2-(isopropoxycarbonyl)ethyl and 2-(tert-butoxycarbonyl)ethyl.

The term "C₁₋₆alkyl" as used herein, means a straight or branched chain hydrocarbon containing from 1 to 6 carbon atoms. Representative examples of alkyl include, but are not limited to methyl, ethyl, n-propyl, iso-propyl, n-butyl, sec-butyl, iso-butyl, tert-butyl, n-pentyl, isopentyl, neopentyl and n-hexyl.

The term "C₁₋₆alkylcarbonyl" as used herein, means a C₁₋₆alkyl group appended to the parent molecular moiety through a carbonyl group, as defined herein. Representative examples of C₁₋₆alkylcarbonyl include but are not limited to propionyl, 2,2-dimethylpropionyl, butanoyl and pentanoyl.

The term "C₁₋₆alkylcarbonyloxy" as used herein, means a C₁₋₆alkylcarbonyl group, as defined herein, appended to the parent molecular moiety through an oxy moiety, as deemed herein. Representative examples of C₁₋₆alkylcarbonyloxy include, but are not limited to acetoxy, propanoyloxy and 2,2-dimethylpropanoyloxy.

The term "C₁₋₆alkylthio" as used herein, means a C₁₋₆alkyl group appended to the parent molecular moiety through a sulfur atom. Representative examples of C₁₋₆alkylthio include, but are not limited, methylthio, ethylthio, tert-butylthio and hexylthio.

The term "C₂₋₆alkynyl" as used herein, means a straight or branched chain hydrocarbon group containing from 2 to 6 carbon atoms, preferably 2 to 4 carbon atoms, preferably in a straight chain, and containing at least one carbon-carbon triple bond. Representative examples of a C₂₋₆alkynyl include, but are not limited, to acetylenyl, 1-propynyl, 2-propynyl, 3-butynyl, 2-pentynyl, 3-methyl-1-pentynyl and 1-butynyl.

The term "C₃₋₆cycloalkyl" as used herein means a cyclic C₃₋₆alkyl as defined herein. Representative examples of a C₃₋₆cycloalkyl include cyclopropyl, cyclobutyl, cyclopentyl and cyclohexyl.

The term "C₃₋₆cycloalkylC₁₋₄alkyl" as used herein means a cyclic C₃₋₆alkyl group appended to the parent molecular moiety through a C₁₋₄alkyl. Representative examples of C₃₋₆cycloalkylC₁₋₄alkyl include cyclopropylmethyl and cyclopentylmethyl.

The term C₃₋₆cycloalkylC₁₋₄alkoxy as used herein means a cyclic C₃₋₆alkyl group appended to the parent molecular moiety through a C₁₋₄alkoxy. Representative examples of C₃₋₆cycloalkylC₁₋₄alkoxy include cyclopropyl-methoxy, cyclobutyl-methoxy, cyclopentyl-methoxy, cyclopropyl-ethoxy and cyclobutyl-ethoxy.

The term "carbonyl" as used herein, means a -C(O)- group.

The term "carboxy" as used herein, means a -CO₂ group.

The term "carboxyC₁₋₆alkyl " as used herein, means a carboxyl group appended to the parent molecular moiety through a C₁₋₆alkyl group, as defined herein. Representative examples of carboxyC₁₋₆alkyl include, but are not limited to, carboxymethyl, 2-carboxyethyl and 3-carboxypropyl.

The term "cyano" as used herein, means a -CN group.

The term "cyanoC₁₋₆alkyl" as used herein, means a cyano group, as defined herein, appended to the parent molecular moiety through a C₁₋₆alkyl group, as defined herein. Representative examples of cyanoC₁₋₆alkyl include, but are not limited to cyanomethyl, 2-cyanoethyl and 3-cyanopropyl.

The term "formyl" as used herein, means a -C(O)H group.

The term "formymC₁₋₆alkyl" as used herein, means a formyl group, as defined herein, appended to the parent molecular moiety through a C₁₋₆alkyl group, as defined herein. Representative examples of formylC₁₋₆alkyl include, but are not limited to, 2-oxoethyl and 3-oxopropyl

The term "halo" or "halogen" as used herein, means -F, -Cl, -Br or -I.

The term "haloC₁₋₆alkoxy" as used herein, means at least one halogen, as defined herein, appended to the parent molecular moiety through a C₁₋₆alkoxy group, as defined herein. Representative examples of haloC₁₋₆alkoxy include, but are not limited to, chloromethoxy, 2-fluoroethoxy, 2,2-difluoro-ethoxy, trifluoromethoxy, 1,2-difluoroethoxy, 2,2,2-trifluoro-ethoxy and pentafluoroethoxy.

The term "haloC₁₋₆alkyl" as used herein, means at least one halogen, as defined herein, appended to the parent molecular moiety through a C₁₋₆alkyl group, as defined herein. Representative examples of haloC₁₋₆alkyl include, but are not limited to, chloromethyl, 2-fluoroethyl, trifluoromethyl, pentafluoroethyl and 2-chloro-3-fluoropentyl.

The term haloC₃₋₆cyloalkylC₁₋₄alkyl as used herein, means a C₃₋₆cycloalkylC₁₋₄alkyl as defined hereabove wherein said C₃₋₆cycloalkylC₁₋₄alkyl is substituted at any position on the carbocyclic ring or C₁₋₄alkyl chain by from one to four halo atoms, preferably by 1, 2 or 3 fluorine atoms.

The term haloC₃₋₆cycloalkylC₁₋₄alkoxy as used herein, means a C₃₋₆cycloalkylC₁₋₄alkyl as defined hereabove wherein said C₃₋₆cycloalkylC₁₋₄alkoxy is substituted at any position on the carbocyclic ring or alkyl chain of the C₁₋₄alkoxy by from one to four halo atoms, preferably by 1, 2 or 3 fluorine atoms.

The term "hydroxy" as used herein, means an -OH group.

The term "hydroxyC₁₋₆alkyl" as used herein, means at least one hydroxy group, as defined herein, appended to the parent molecular moiety through a C₁₋₆alkyl group, as defined herein. Representative examples of hydroxyC₁₋₆alkyl include, but are not limited to methanol, propanol and propan-1,2-diol.

The term "mercapto" as used herein, means a -SH group.

The term "mercaptoC₁₋₆alkyl" as used herein, means at least one mercapto group, as defined herein, appended to the parent molecular moiety through a C₁₋₆alkyl group, as defined herein. Representative examples of mercaptoC₁₋₆alkyl include, but are not limited to methane thiol, ethane thiol and propane thiol.

The term "-NR³R⁴" as used herein, means two groups, R³ and R⁴, which are appended to the parent molecular moiety through a nitrogen atom. R³ and R⁴are independently selected from hydrogen, C₁₋₄alkyl, C₁₋₄alkylcarbonyl as defined herein. Representative examples of - NR³R⁴ include, but are not limited to acetamido, amino, methylamino, dimethylamino and ethylamino.

The term "-C₁₋₄alkyl(NR³R⁴)" as used herein, means a - NR³R⁴ as defined herein, appended to the parent molecular moiety through an C₁₋₄alkyl group, as defined herein. Representative examples of -C₁₋₄alkyl (NR³R⁴) include, but are not limited, aminomethyl, (methylamino)methyl, 2-aminoethyl and (dimethylamino)methyl.

The term "-CONR³R⁴" as used herein, means a -(NR³R⁴)group, as defined herein, appended to the parent molecular moiety through a carbonyl group, as defined herein. Representative examples of -CONR³R⁴ include, but are not limited to carboxamido, N,N-dimethyl carboxamido, N-methyl carboxamido and N-ethylcarboxamido.

The term "-C₁₋₄alkylCO(NR³R⁴) as used herein, means a -CONR³R⁴ group, as defined herein, appended to the parent molecular moiety through an C₁₋₄alkyl group, as defined herein. Representative examples of -C₁-₄alkylCO(NR³R⁴) include, but are not limited to carboxamidoethyl, N-methyl carboxamidoethyl, carboxamidobutyl and N,N-dimethyl carboxamido butyl.

The term "-SO₂NR³R⁴" as used herein, means a -NR³R⁴ group, as defined herein, appended to the parent molecular moiety through a sulfonyl group, as defined herein. Representative examples of -SO₂NR³R⁴ include, but are not limited to sulfonamido, N-methyl N,N-dimethylsulfonamido, N-methylsulfonamide and N-ethylsulfonamido.

The term "nitro" as used herein, means a -NO₂ group.

The term "nitrogen protecting group" or "N-protecting group" as used herein, means those groups intended to protect an amino group against undesirable reactions during synthetic procedures. Nitrogen protecting groups comprise carbamates, amides, N-benzyl derivatives, and imine derivatives. Preferred nitrogen protecting groups benzyl, trityl, acyl or carbamates of t-butyl, benzyl, 2,4-dichlorobenzyl, 2-(biphenylyl)isopropyl, 9-fluorenylmethyl, isonicotinyl or allyl.

The term "oxo" as used herein, means a C=O moiety.

The term "oxy" as used herein, means a -O- moiety.

The term "phenoxy" as used herein, means a phenyl group appended to the parent molecule through an oxy moiety as defined herein.

The term "phenyl(CH₂)ₙO wherein n=0, 1 or 2" as used herein, means a phenyl group appended to the parent molecule through an oxy or alkoxy moiety wherein said alkoxy moiety has one or two carbon atoms.

The term "sulfonyl" as used herein, means a -SO₂- group.

----- Represents a bond that exists as either a single or a double bond.

In a preferred embodiment the compounds of the present invention comprise compounds according to formula I as described above wherein A is a phenyl optionally substituted with one, two, three or four substituents independently selected from C₂₋₆alkenyl, C₁₋₆ alkoxy, C₁₋₆alkoxyC₁₋₄alkoxy, C₁₋₆alkoxyC₁₋₄alkyl, C₁₋₆ alkoxycarbonyl, C₁₋₆alkoxycarbonylC₁₋₄alkyl, C₁₋₆alkyl, C₁₋₆alkylcarbonyl, C₁₋₆alkylcarbonyloxy, C₁₋₆alkylthio, C₂₋₆alkynyl, carboxy, carboxyC₁₋₆alkyl, cyano, cyanoC₁₋₆alkyl, C₃₋₆cycloalkyl, C₃₋₆cycloalkylC₁₋₄alkyl, formyl, formylC₁₋₆alkyl, haloC₁₋₆alkoxy, haloC₁₋₆alkyl, halogen, hydroxy, hydroxyC₁₋₆alkyl, mercapto, mercaptoC₁₋₆alkyl, nitro, triphenylmethyl (trityl), furyl, thienyl, pyrrolyl, pyrazolyl, imidazolyl, triazolyl, oxazolyl, isoxazolyl, thiazolyl, thiadiazolyl, isothiazolyl, oxadiazolyl, oxatriazolyl, pyridinyl, pyridazinyl, pyrimidinyl, pyrazinyl, triazinyl, tetrazolyl, -C(NNR³R⁴, -NR³R⁴, -C₁₋₄alkyl (NR³R⁴), -CO(NR³R⁴), -C₁₋₄alkylCO(NR³R⁴), -S(O)₂ NR³R⁴, -NR⁵S(O)₂ R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂ R⁵, phenyl and phenyl(CH₂)ₙO, wherein said phenyl or phenyl(CH₂)ₙO is optionally substituted with one, two or three substituents independently selected from chlorine, fluorine and cyano; or where two substituents on adjacent carbon atoms of said aryl together optionally represent -OCH₂CH₂-, -OCH₂CH(OH)-, -OCH₂CH₂O- or -OCH₂O-.

In a further embodiment the present invention comprises compounds according to formula I, wherein R¹ is H, C₁₋₃alkoxycarbonyl, C₁₋₃alkyl, cyanoC₁₋₃alkyl, hydroxy or hydroxyC₁₋₃alkyl; A is a phenyl optionally substituted with one, two or three substituents independently selected from amide, C₂₋₄alkenyl, C₁₋₃alkoxy, C₁₋₃alkoxyC₁₋₃alkyl, C₁₋₃alkoxycarbonyl, C₁₋₃alkyl, C₁₋₃alkylcarbonyl, C₁₋₃alkylcarbonyloxy, C₁₋₃alkylthio, C₂₋₄alkynyl, carboxy, carboxyC₁₋₃alkyl, cyano, cyanoC₁₋₃alkyl, formyl, formylC₁₋₃alkyl , haloC₁₋₃alkoxy, haloC₁₋₃alkyl, halogen, hydroxy, hydroxyC₁₋₃alkyl, mercapto, mercaptoC₁₋₃alkyl, nitro, furyl, thienyl, pyrrolyl, pyrazolyl, imidazolyl, triazolyl, oxazolyl, isoxazolyl, thiazolyl, thiadiazolyl, isothiazolyl, oxadiazolyl, oxatriazolyl, pyridinyl, pyridazinyl, pyrimidinyl, pyrazinyl, triazinyl, tetrazolyl, phenyl and phenyl(CH₂)ₙO, wherein said phenyl or phenyl(CH₂)ₙO is optionally substituted with one, two or three substituents independently selected from chlorine, fluorine and cyano, wherein n=0, 1 or 2.

Preferably compounds of the present invention have a formula according to formula I wherein R² is hydrogen or absent. Most preferably in compounds according to the invention R² is absent.

Preferably compounds of the present invention have a formula according to formula I wherein R¹ is selected from hydrogen, C₁₋₆alkyl and hydroxyl, more preferably R¹ is selected from hydrogen and C₁₋₃alkyl. Further preferrred R¹ is selected from hydrogen and methyl. Most preferably R¹ is hydrogen.

. In a preferred embodiment, the invention is directed to compounds of the fomula I wherein R¹ is H, C₁₋₃alkyl or hydroxy; A is a phenyl optionally substituted with one, two or three substituents independently selected from amide, C₂₋₄alkenyl, C₁₋₃alkoxy, C₁₋₃alkoxyC₁₋₃alkyl, C₁₋₃alkoxycarbonyl, C₁₋₃alkyl, C₁₋₃alkylcarbonyl, C₁₋₃alkylcarbonyloxy, C₁₋₃alkylthio, C₂₋₄alkynyl, carboxy, carboxyC₁₋₃alkyl, cyano, cyanoC₁₋₃alkyl, formyl, formylC₁₋₃alkyl, haloC₁₋₃alkoxy, haloC₁₋₃alkyl, halogen, hydroxy, hydroxyC₁₋₃alkyl, mercapto, mercaptoC₁₋₃alkyl, nitro, furyl, thienyl, pyrrolyl, pyrazolyl, imidazolyl, triazolyl, oxazolyl, isoxazolyl, thiazolyl, thiadiazolyl, isothiazolyl, oxadiazolyl, oxatriazolyl, pyridinyl, pyridazinyl, pyrimidinyl, pyrazinyl, triazinyl, tetrazolyl, phenyl and phenyl(CH₂)ₙO, wherein said phenyl or phenyl(CH₂)ₙO is optionally substituted with one, two or three substituents independently selected from chlorine, fluorine and cyano, wherein n=0, 1 or 2.

In a further preferred embodiment compounds of the invention have a formula according to formula I wherein R¹ is H or methyl; A is a phenyl optionally substituted with one, two or three substituents independently selected from cyano, fluorine, chlorine, bromine, methyl, ethyl, isopropyl, methoxy, ethoxy, isopropyloxy, benzyloxy, trifluoromethyl, difluoromethoxy, trifluoromethoxy, phenoxy, 4-chlorophenoxy, 4-cyanophenoxy and 4-fluorophenoxy.

In a compound according to formula I, A is preferably a phenyl group optionally substituted with one, two or three substituents independently selected from cyano, fluorine, chlorine, bromine, methyl, ethyl, isopropyl, methoxy, ethoxy, benzyloxy, isopropyloxy, trifluoromethyl, difluoromethoxy, trifluoromethoxy, phenoxy, 4-chlorophenoxy, 4-cyanophenoxy or 4-fluorophenoxy. Most preferred substituted phenyl groups represented by A are selected from 2-fluorophenyl, 3-fluorophenyl, 3,5-difluorophenyl, 3,4-difluorophenyl, 3,4,5-trifluorophenyl, 3-cyanophenyl and 3-methoxyphenyl.

The applicants have found that selectivity for the α4β2 nicotinic receptor as compared to the α4β2 nicotinic receptor and/or potency can be enhanced where A is a phenyl group which has two substituents at the 3 and 5 positions of the phenyl ring. The present invention therefore further relates to a compound according to formula III or a pharmaceutically acceptable salt thereof: 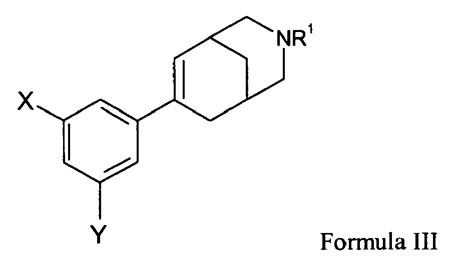 wherein X is selected from a C₁₋₆alkyl, C₂₋₆alkenyl, C₁₋₆alkoxy, C₁₋₆alkoxyC₁₋₄alkoxy, C₁₋₆alkoxyC₁₋₄alkyl, C₁₋₆alkoxycarbonyl, C₁₋₆alkoxycarbonylC₁₋₄alkyl, C₁₋₆alkylcarbonyl, C₁₋₆alkylcarbonyloxy, C₁₋₆alkylthio, C₂₋₆alkynyl, carboxy, carboxyC₁₋₆alkyl, cyano, cyanoC₁₋₆alkyl, C₃₋₆cycloalkyl, C₃₋₆CycloalkylC₁₋₄alkyl, C₃₋₆cycloalkylC₁₋₄alkoxy, wherein said C₃₋₆cycloalkylC₁₋₄alkyl or C₃₋₆cycloalkylC₁₋₄alkoxy is optionally substituted with 1 to 3 halogen atoms; formylC₁₋₆alkyl, haloC₁₋₆alkoxy, haloC₁₋₆alkyl, halogen, hydrogen, hydroxy, hydroxyC₁₋₆alkyl, carboxy, mercapto, mercaptoC₁₋₆alkyl, nitro, triphenylmethyl (trityl), -C(NH)NR³R⁴, -NR³R⁴, -C₁₋₄alkyl (NR³R⁴), -CO(NR³R⁴), -C₁₋₄alkylCO(NR³R⁴), -S(O)₂ NR³R⁴, -NR⁵S(O)₂ R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂O_{R}5, -S(O)₂ R⁵, a heteroaryl optionally substituted by one, two or three methyl substituents, wherein said heteroaryl is selected from furyl, thienyl, pyrrolyl, pyrazolyl, imidazolyl, triazolyl, oxazolyl, isoxazolyl, thiazolyl, thiadiazolyl, isothiazolyl, oxadiazolyl, oxatriazolyl, pyridinyl, pyridazinyl, pyrimidinyl, pyrazinyl, triazinyl and tetrazolyl, phenyl and phenyl(CH₂)ₙO, wherein said phenyl or phenyl(CH₂)ₙO is optionally substituted with one, two or three substituents independently selected from chlorine, fluorine, cyano, C₁₋₃alkyl and C₁₋₃alkoxy wherein said C₁₋₃alkyl or C₁₋₃alkoxy is optionally substituted with 1 to 3 fluorines; Y is selected from cyano, hydrogen, fluoro, chloro and bromo; R¹ is selected from hydrogen and C₁₋₄alkyl; R³ and R⁴ are independently selected from hydrogen, C₁₋₄alkyl and C₁₋₄alkylcarbonyl; R⁵, R⁶ and R⁷ are independently selected from hydrogen, C₁₋₄alkyl, phenyl and phenylC₁₋₄alkyl, n= 0,1 or 2.

Preferably in this embodiment, X is selected from C₁₋₆alkyl, C₁₋₆alkoxy, C₁₋₆alkoxyC₁₋₄alkyl, C₁₋₆alkylcarbonyl, C₁₋₆alkylthio, C₃₋₆cycloalkylC₁₋₄alkoxy, haloC₁₋₆alkoxy, haloC₁₋₆alkyl, halogen, hydrogen, hydroxy, mercapto, -CO(NR³R⁴), -C₁₋₄alkylCO(NR³R⁴), a heteroaryl optionally substituted by one, two or three methyl substituents, wherein said heteroaryl is selected from furyl, thienyl, pyrrolyl, pyrazolyl, imidazolyl, triazolyl, oxazolyl, isoxazolyl, thiazolyl, thiadiazolyl, isothiazolyl, oxadiazolyl, oxatriazolyl, pyridinyl, pyridazinyl, pyrimidinyl, pyrazinyl, triazinyl and tetrazolyl; Y is selected from cyano, hydrogen, fluoro and chloro; R¹ is hydrogen or a methyl; R³ and R⁴are independently selected from hydrogen and methyl.

In another preferred embodiment the invention relates to compounds of the fomula III wherein X is selected from C₁₋₃alkyl, C₂₋₄alkenyl, C₁₋₃alkoxy, C₁₋₃alkoxyC₁₋₃alkoxy, C₁₋₃alkoxyC₁₋₃alkyl, C₁₋₃alkoxycarbonyl, C₁₋₃alkoxycarbonylC₁₋₃alkyl, C₁₋₃alkylcarbonyl, C₁₋₃alkylcarbonyloxy, C₁₋₃alkylthio, C₂₋₄alkynyl, carboxy, cyano, cyanoC₁₋₃alkyl, cyclopropyl, cyclopropylC₁₋₃akyl, cyclopropylC₁₋₃alkoxy, wherein said C₁₋₃alkyl, C₁₋₃alkoxy, cyclopropylC₁₋₃alkyl, cyclopropylC₁₋₃alkoxy is optionally substituted with 1 to 3 halogen atoms independently selected from fluorine and chlorine which may be at any position on the carbocyclic ring and/or the C₁₋₃alkyl chain; formylC₁₋₃alkyl, halogen, hydrogen, hydroxy, hydroxyC₁₋₃alkyl, mercapto, mercaptoC₁₋₃alkyl, nitro, -C(NH)NR³R⁴, -NR³R⁴, -C₁₋₃alkyl (NR³R⁴), -CO(NR³R⁴), -C₁₋₃akylCO(NR³R⁴), -S(O)₂ NR³R⁴,-NR⁵S(O)₂ R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵ -S(O)₂ R⁵, a heteroaryl optionally substituted by one, two or three methyl substituents, wherein said heteroaryl is selected from furyl, thienyl, pyrrolyl, pyrazolyl, imidazolyl, triazolyl, oxazolyl, isoxazolyl, thiazolyl, thiadiazolyl, isothiazolyl, oxadiazolyl, pyridinyl, pyridazinyl, pyrimidinyl and pyrazinyl, phenyl and phenyl(CH₂)ₙO, wherein said phenyl or phenyl(CH₂)ₙO is optionally substituted with one, two or three substituents independently selected from fluorine, cyano, methyl, ethyl, methoxy and ethoxy, wherein said methyl, ethyl, methoxy or ethoxy is optionally substituted with 1 to 3 fluorines; Y is selected from cyano, hydrogen, fluoro and chloro; R¹ is selected from methyl and hydrogen; R³ and R⁴ are independently selected from hydrogen, C₁₋₃alkyl and C₁₋₃alkylcarbonyl; R⁵, R⁶ and R⁷ are independently selected from hydrogen and C₁₋₃alkyl; and n is 0 or 1.

Preferably in this embodiment X is selected from C₁₋₃alkyl, C₁₋₃alkoxy, C₁₋₃atkoxyC₁₋₃alkyl, C₁₋₃alkoxycarbonyl, C₁₋₃alkylcarbonyl, C₁₋₃alkylcarbonyloxy, C₁₋₃alkylthio, carboxy, cyano, cyanoC₁₋₃atkyl, cyclopropyl, cyclopropylC₁₋₃alkyl and cyclopropylC₁₋₃alkoxy, wherein said C₁₋₃alkyl, C₁₋₃alkoxy, cyclopropylC₁₋₃alkyl or cyclopropylC₁₋₃alkoxy is optionally substituted with 1 to 3 halogen atoms independently selected from fluorine and chlorine which may be at any position on the carbocyclic ring and/or the C₁₋₃alkyl chain, halogen, hydrogen, hydroxy, mercapto, -NR³R⁴, -C₁₋₃alkyl(NR³R⁴), -CO(NR³R⁴), -C₁₋₃alkylCO(NR³R⁴), -S(O)₂ R⁵, a heteroaryl optionally substituted by one, two or three methyl substituents, wherein said heteroaryl is selected from furyl, thienyl, pyrrolyl, pyrazolyl, imidazolyl, triazolyl, oxazolyl, isoxazolyl, thiazolyl, thiadiazolyl, isothiazolyl, oxadiazolyl, pyridinyl, pyridazinyl, pyrimidinyl and pyrazinyl; wherein Y is selected from cyano, hydrogen, fluoro and chloro; R¹ is hydrogen or methyl; R³ and R⁴ are independently selected from hydrogen and C₁₋₃alkyl; and R⁵ is selected from hydrogen and C₁₋₃alkyl.

Where X is a heteroaryl group this is preferably selected from furyl, thienyl, pyrrolyl, pyrazolyl, imidazolyl, triazolyl, oxazolyl, isoxazolyl, thiazolyl, thiadiazolyl, isothiazolyl, oxadiazolyl, pyridinyl, pyridazinyl, pyrimidinyl and pyrazinyl; more preferably selected from furyl, pyrrolyl, imidazolyl, triazolyl, isoxazolyl, oxadiazolyl, thiadiazolyl and pyridinyl, wherein said heteroaryl is optionally substituted by one, two or three methyl substituents; wherein Y is cyano, hydrogen or fluoro and R¹ is hydrogen Where heteroaryl group X is substituted with a methyl, this is preferably a single methyl substitution.

In a compound accoriding to formula III, Y can be selected from cyano, hydrogen, fluoro and chloro. Preferably Y is selected from cyano, hydrogen and fluoro. More preferably Y is cyano or fluoro. In a most preferred embodiment of the invention Y is fluoro.

Selectivity for the α4β2 nicotinic receptor as compared to the α4β2 nicotinic receptor may be particularly enhanced and/or potency at the α4β2 may be particularly increased in compounds according to formula III, wherein X is selected from hydrogen, fluoro, chloro, bromo, -CONH₂, acetyl, C₁₋₃alkyl, C₁₋₃alkoxy, cyclopropylC₁₋₃alkyl, cyclopropylC₁₋₃alkoxy, wherein said C₁₋₃alkyl, C₁₋₃alkoxy, cyclopropylC₁₋₃alkyl, cyclopropylC₁₋₃alkoxy is optionally substituted with 1 to 3 fluorines which may be at any position on the cyclopropyl ring and/or the C₁₋₃alkyl chain, a heteroaryl optionally substituted with a methyl wherein said heteroaryl is selected from furyl, pyrrolyl, imidazolyl, triazolyl, isoxazolyl, oxadiazolyl, thiadiazolyl and pyridinyl.

A particularly preferred embodiment of the invention therefore relates to compounds according to formula III, wherein X is selected from hydrogen, fluoro, chloro, bromo, -CONH₂, acetyl, C₁₋₃alkyl, C₁₋₃alkoxy, cyclopropylC₁₋₃alkyl, cyclopropylC₁₋₃alkoxy, wherein said C₁₋₃alkyl, C₁₋₃alkoxy, cyclopropylC₁₋₃alkyl, cyclopropylC₁₋₃alkoxy is optionally substituted with 1 to 3 fluorines which may be at any position on the cyclopropyl ring and/or the C₁₋₃alkyl chain, a heteroaryl optionally substituted with a methyl wherein said heteroaryl is selected from furyl, pyrrolyl, imidazolyl, triazolyl, isoxazolyl, oxadiazolyl, thiadiazolyl and pyridinyl; Y is selected from cyano, hydrogen and fluoro and wherein X and Y do not equal hydrogen; R¹ is methyl or hydrogen, preferably hydrogen.

In a further preferred embodiment compounds can be described in relation to formula III, wherein X is selected from fluoro, methoxy, ethoxy, acetyl, -CONH₂, 2-fluoro-ethoxy, 2,2-difluoro-ethoxy, 2,2,2-trifluoro-ethoxy, cyclopropyl-methoxy, a heteroaryl optionally substituted with a methyl wherein said heteroaryl is selected from 1-imidazolyl, 5-isoxazolyl, 3-pyridinyl, 4-pyridinyl and 5-thiadiazolyl.

In a most preferred embodiment the invention relates to compounds according to formula III, wherein X is selected from fluoro, methoxy, ethoxy, acetyl, -CONH₂, 2-fluoro-ethoxy, 2,2-difluoro-ethoxy, 2,2,2-trifluoro-ethoxy, cyclopropyl-methoxy, a heteroaryl optionally substituted with a methyl wherein said heteroaryl is selected from 1-imidazolyl, 5-isoxazolyl, 3-pyridinyl, 4-pyridinyl and 5-thiadiazolyl; Y is preferably fluoro or cyano, most preferably fluoro and R¹ is hydrogen.

The present invention contemplates stereoisomers and mixtures thereof that are specifically included within the scope of this invention. Stereoisomers include enantiomers, diastereomers, and mixtures of enantiomers or diastereomers. Individual stereoisomers of compounds of the present invention may be prepared synthetically from commercially available starting materials that contain asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary or (2) direct separation of the mixture of optical enantiomers on chiral chromatographic columns.

For use in medicine, the salts of the compounds of formula I or fomula III will be non-toxic pharmaceutically acceptable salts. Other salts may, however, be useful in the preparation of the compounds according to the invention or of their non-toxic pharmaceutically acceptable salts. Suitable pharmaceutically acceptable salts of the compounds of this invention include acid addition salts which may, for example, be formed by mixing a solution of the compound according to the invention with a solution of a suitable acid such as hydrochloric acid, fumaric acid, p-toluenesulphonic acid, maleic acid, succinic acid, acetic acid, citric acid, tartaric acid, carbonic acid, phosphoric acid or sulphuric acid. Salts of amine groups may also comprise quaternary ammonium salts in which the amino nitrogen atom carries a suitable organic group such as an alkyl, alkenyl, alkynyl or aralkyl moiety. Furthermore, where the compounds of the invention carry an acidic moiety, suitable pharmaceutically acceptable salts thereof may include metal salts such as alkali metal salts, e.g. sodium or potassium salts; and alkaline earth metal salts, e.g. calcium or magnesium salts.

The salts may be formed by conventional means, such as by reacting the free base form of the product with one or more equivalents of the appropriate acid in a solvent or medium in which the salt is insoluble, or in a solvent such as water which is removed *in vacuo* or by freeze drying or by exchanging the anions of an existing salt for another anion on a suitable ion exchange resin.

Preferably the compositions according to the invention are in unit dosage forms such as tablets, pills, capsules, wafers, powders, granules, solutions or suspensions, or suppositories, for oral, parenteral or rectal administration, or administration by inhalation or insufflation. Oral compositions such as tablets, pills, capsules or wafers are particularly preferred.

The present invention further provides a process for the preparation of a pharmaceutical composition comprising a compound of formula I or formula III, which process comprises bringing a compound of formula I or formula III into association with a pharmaceutically acceptable carrier or excipient.

The excellent pharmacological profile of the compounds of the present invention offers the opportunity for their use in therapy at low doses thereby minimising the risk of unwanted side effects.

Effective amounts of a compound in accordance with the present invention can be used in the manufacture of a medicament for the treatment of one or more conditions selected from inflammatory bowel disease, ulcerative colitis, pyoderma gangrenosum, Crohn's disease, irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotrophic lateral sclerosis (ALS), cognitive dysfunction, hypertension, bulimia, anorexia, obesity, cardiac arrhythmias, gastric acid hypersecretion, ulcers, pheochromocytoma, progressive supranuclear palsy, chemical dependencies and addictions, dependencies on, or addictions to nicotine (or tobacco products), alcohol, benzodiazopines, barbiturates, opioids or cocaine, headache, migraine, stroke, traumatic brain injury (TBI), obsessive-compulsive disorder (OCD), psychosis, Huntington's chorea, tardive dyskinesia, hyperkinesia, dyslexia, schizophrenia, multi-infarct dementia, age-related cognitive decline, epilepsy, petit mal absence epilepsy, senile dementia of the Alzheimer's type (AD), Parkinson's disease (PD), attention deficit hyperactivity disorder (ADHD), attention deficit disorder (ADD), restless legs syndrome (RLS), mild cognitive impairment, cognitive enhancement in schizophrenia, drug induced extrapyramidal symptoms, conduct disorder, oppositional defiant disorder, anxiety in anxious smokers, cardiovascular risk in pregnancy, delayed ejaculation, emesis, diarrhoea, nicotine gum addiction, sleep prevention, ischemia, and Tourette's Syndrome in a mammal, preferably a human.

In a preferred embodiment the invention relates to the use of an effective amount of a compound, as presented hereabove, in the manufacture of a medicament for the treatment or prevention of one or more human conditions selected from chronic pain, acute pain, anxiety, panic disorder, depression, bipolar disorder, amyotrophic lateral sclerosis (ALS), cognitive dysfunction, hypertension, bulimia, anorexia, obesity, cardiac arrhythmias, headache, migraine, stroke, traumatic brain injury (TBI), obsessive-compulsive disorder (OCD), psychosis, Huntington's chorea, tardive dyskinesia, hyperkinesia, dyslexia, schizophrenia, multi- infarct dementia, age-related cognitive decline, epilepsy, petit mal absence epilepsy, senile dementia of the Alzheimer's type (AD), Parkinson's disease (PD), attention deficit hyperactivity disorder (ADHD), attention deficit disorder (ADD), mild cognitive impairment, cognitive enhancement in schizophrenia, ischemia, and Tourette's Syndrome. Further preferred is a use in the treatment or prevention of chronic pain or acute pain.

The abbreviations that have been used in the Schemes and examples that follow are as follows: NH₄OH:35% ammonium hydroxide solution; Brine: saturated aqueous sodium chloride; CHCl₃: chloroform; DCM: dichloromethane; DIBAL^{®}: diisobutylaluminiumhydride; DME: 1,2-dimethoxyethane; DMF: N,N-dimethylformamide; DMSO: dimethyl sulfoxide; Et₃N: triethylamine; Et₂O: diethyl ether; EtOAc: ethyl acetate; EtOH: ethanol; HCl: hydrochloric acid; IPA: isopropyl alcohol; K₂CO₃: potassium carbonate; LDA: lithium diisopropylamide; LiHMDS: lithium hexamethyldisilazide; MeOH: methanol; MgSO₄: magnesium sulfate; Na₂CO₃: sodium carbonate; NaHCO₃: sodium hydrogen carbonate; NaOH: sodium hydroxide; NH₃: ammonia; Pd(OH)₂: palladium (II) hydroxide; PTSA: 4-toluenesulfonic acid monohydrate; SCX-2 cartridge^{®}: SiO₂ impregnated with benzene sulfonic acid in a plastic cartridge; SiO₂: silica gel; TEMPO^{®}: 2,2,6,6-Tetramethyl-1-piperidinooxy free radical; THF: tetrahydrofuran; Tlc: thin layer chromatography; TFA: trifluoroacetic acid.

The compounds according to the subject invention, as depicted generally in formula I or fomula III, can be prepared by a variety of procedures and synthetic routes. Representative procedures and synthetic routes are shown in, but not limited to, the following reaction Schemes I through IV. Isolation and purification of the products is accomplished by standard procedures known to a chemist skilled in the art. 'Inert solvent' refers to a solvent system in which the reaction components do not interact with the starting materials, reagents or intermediates of products in a manner which would adversely affect the outcome of the reaction. During any of the following synthetic sequences it may be necessary and/or desirable to protect sensitive or reactive groups on any of the molecules concerned. This may be achieved by means of conventional protecting groups, such as those described in T W Greene and P G M Wuts, Protective groups in Organic Synthesis, John Wiley & Sons, 3rd edition 1999.

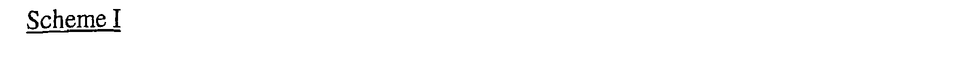

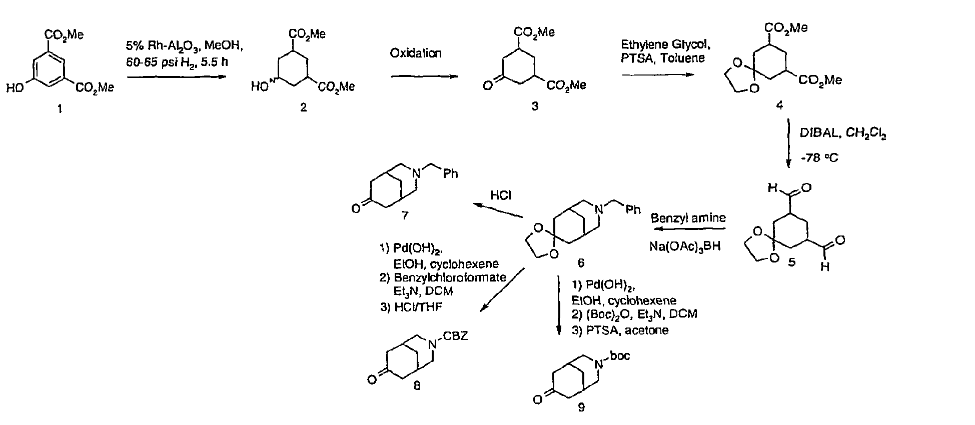

Referring to Scheme I, hydrogenation of dimethyl 5-hydroxyisophthalate by a known method (W J Gensler and P H Solomon J. Org. Chem. 1973, 38(9), 1726-1731) provides access to 5-hydroxy-cyclohexane-1,3-dicarboxylic acid dimethyl ester. Oxidation (Swern or TEMPO^{®} conditions), protection of the resulting ketone, reduction (DIBAL^{®}) to the dialdehyde, followed by reductive amination with benzyl amine, provides a precursor in which the protecting group on the nitrogen may be manipulated or maintained as benzyl. A typical Swern reaction involves the action of a solution of oxalyl chloride in a suitable solvent (such as dry DCM) on a solution of dry DMSO in dry DCM at low temperature (generally around -60°C), on dimethyl 5-hydroxycyclohexane-1,3-dicarboxylate. The reaction is allowed to warm to 0°C prior to a base quench (preferably with Et₃N), and standard aqueous workup to provides compound 3. Alternatively, compound 3 can be prepared by the oxidation of dimethyl 5-hydroxycyclohexane-1,3-dicarboxylate with aqueous potassium bromide and TEMPO^{®} at low temperature (preferably at -5°C), under a nitrogen atmosphere in DCM as the preferred solvent, followed by a careful addition of a slurry of sodium hydrogen carbonate in sodium hypochlorite solution (5% free chlorine), again at low temperature (between - 5°C and 5°C). The reaction is allowed to warm to room temperature and is then treated with IPA. Standard extraction methods give compound 3. The ketone functionality is then masked as a cyclic ketal 4, utilising the standard reaction conditions of ethylene glycol in the presence of a catalytic amount of acid (preferably PTSA), in toluene under reflux in the presence of a Dean-Stark apparatus. Reduction of 4 to the dialdehyde 5 can be achieved by use of a suitable reducing agent, such as DIBAL^{®} (preferably as a solution in toluene). The reaction is performed in a suitable inert solvent (such as DCM) at low temperature (generally in the range of -60 to -78°C). Compound 5 may then be converted to the amine 6 by means of a reductive amination involving the use of benzylamine in DCM followed by treatment of the intermediate imine with a reducing agent such sodium triacetoxyborohydride at ambient temperature. Conversion to the alternative carbamate protecting groups on the amine functionality of 6 can be achieved by initial removal of the benzyl moiety by catalytic hydrogenation of the benzyl moiety, preferably under transfer hydrogenation conditions, using a 10% solution of cyclohexene in EtOH at reflux in the presence of a suitable catalyst (such as palladium on carbon or palladium hydroxide). Alternatively hydrogenation in a Parr apparatus using a suitable catalyst (such as palladium on carbon or palladium hydroxide) in an alcoholic solvent (such as MeOH or EtOH) may be used. The free amine generated can then be re-protected by another suitable protecting group such as, but not limited to, benzyloxycarbonyl (CBZ) or *t*-butoxycarbonyl (BOC) under standard conditions. The ketal protecting group is removed by treatment with acid in an inert solvent. For compound 7 and 8 the preferred acid is HCl either neat or in THF. For compound 9 the preferred acid is a catalytic amount of PTSA in acetone.

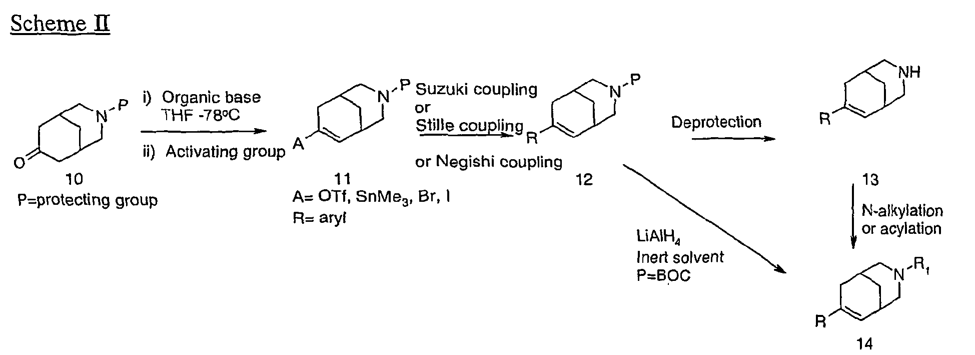

Referring to Scheme II, the ketone 10 can serve as the precursor for metal-mediated coupling methods, such as those of the Stille or Suzuki or Negishi reaction, to generate compounds of general structure 12. Ketone 10 is first converted to an activated intermediate such as a vinyl triflate, vinyl stannane or vinyl halide. The triflate of general formula 11 with A=OTf, can be prepared by the literature method (as described in D J Wustrow and L D Wise Synthesis, 1991, 993) using an organic base (such as LDA or LiHMDS) in a suitable inert solvent (preferably, but not limited to, anhydrous THF). Reaction of compounds of general formula 11 (A= OTf, Br, I) with a variety of aryl boronic acids or esters or in the presence of a base (such as aqueous sodium carbonate, cesium carbonate, sodium hydrogen carbonate or lithium carbonate) and a palladium catalyst (such as, but not limited to, tetrakis(triphenylphosphine)palladium (0); 1,1-bis(diphenylphosphino)ferrocene-palladium (II) dichloride-dichloromethane complex; or palladium (II) acetate and triphenylphosphine) and lithium chloride in an inert solvent (such as DME or THF), generates the amines 12. Alternatively, vinyl stannanes of general formula 11 (A = SnMe₃) may be treated with a palladium catalyst and an aryl halide, or triflate, to provide compounds of general formula 12. Amines of general formula 13 are obtained by removal of the protecting group P by known literature methods (as described in T W Greene and P G M Wuts, Protective groups in Organic Synthesis, John Wiley & Sons, 3rd edition 1999). If the amine is isolated as a salt it may be converted to the corresponding free base by ion exchange chromatography on an SCX-2 cartridge^{®}, eluting with 2M NH₃/MeOH . Alternatively the free base may be obtained by treatment of the salt with a suitable base (including, but not limited to, sodium, lithium and potassium carbonates, bicarbonates and hydroxides), generally in water, to afford compounds of general formula 13 as the free base. Compounds of general formula 13 may be treated with alkylating or acylating reagents and a base (such as, but not limited to, Et₃N) to provide compounds of general formula 14. Alternatively a compound of general formula 12 (where P = BOC) may be directly converted into compounds of general formula 14 (where R₁ = Me) by the action of LiAlH₄ in an inert solvent (such as anhydrous THF or anhydrous Et₂O).

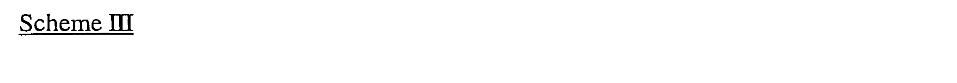

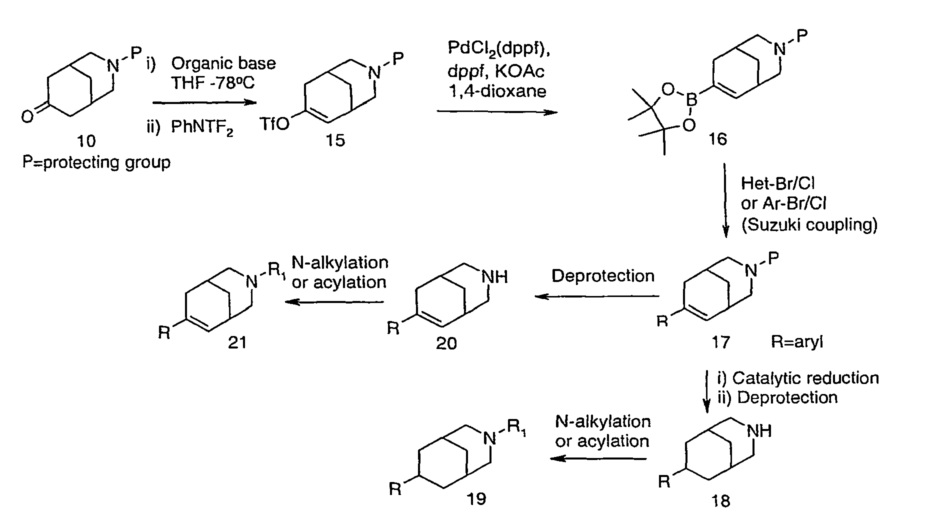

Referring to Scheme III, the ketones 10 can be converted to the corresponding enolates by the action of an organic base (such as LDA or LiHMDS) in an inert solvent (such as THF) at low temperature (generally in the range -60 to -78°C) under a nitrogen atmosphere. The enolates can then be converted to the stable enol triflates 15 by treatment with N-phenyltrifluoromethane sulfonimide at -60 to -78°C, and then allowing the reaction mixture to warm to ambient temperature; followed by isolation utilising standard methods. Conversion to the N-protected cyclic boronates can be achieved by literature methods (P R Eastwood Tetrahedron Letters, 2000, 41 3705) utilising a palladium-mediated cross-coupling with bis(pinacolato)diboron. The preferred palladium catalyst is 1,1-bis(diphenylphosphino)-ferrocene-palladium (II) dichloride-dichloromethane complex in the presence of 1,1-bis(diphenylphosphino)ferrocene and an inorganic base (such as potassium acetate) under a nitrogen atmosphere in an inert solvent (such as 1,4-dioxane) at 80 to 100°C. These boronate esters 16 or the corresponding boronic acids serve as substrates for Suzuki coupling reactions with a wide variety of aromatic and heteroaromatic substrates, to generate compounds of general structure 17. The general reaction conditions involve treatment of 17, in the presence of a palladium catalyst (such as 1,1-bis(diphenylphosphino)ferrocene-palladium (II) dichloride-dichloromethane complex or bis(triphenylphosphine)palladium (II) chloride), with an inorganic base (such as potassium acetate, potassium carbonate or aqueous sodium carbonate) and an aromatic halide or heteroaromatic halide in an inert solvent (such as DMF or THF). Deprotection to the amines of general formulae 18 and 20 can be achieved by the conditions set out in Scheme II. If these amines of general formulae 18 and 20 are isolated as salts these may be converted to the corresponding free bases as described in Scheme II. The alkene functionality present in compounds of general structure 17 can, in some cases, be removed by catalytic hydrogenation in a Parr apparatus, utilizing a palladium catalyst in an alcoholic solvent (such as MeOH or EtOH), prior to removal of the protecting group on the amine functionality. Compounds of general formulae 19 and 21 may be prepared by treatment of the corresponding compounds of general formulae 18 and 20 with alkylating or acylating reagents and a base such as, but not limited to, Et₃N. Alternatively compounds of general formula 17 with P= BOC may be directly converted into compounds of general formula 21 (where R₁ = Me) by the action of lithium aluminium hydride in an inert solvent (such as anhydrous THF or anhydrous Et₂O).

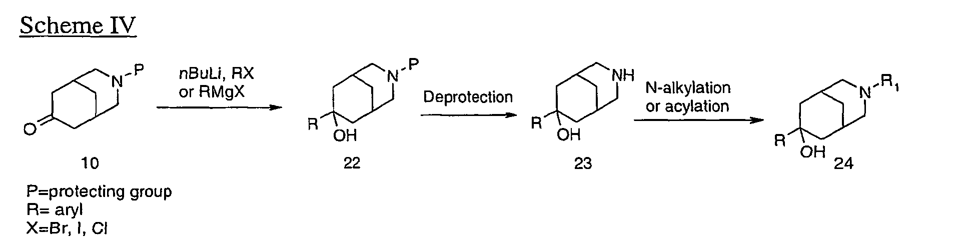

Referring to Scheme IV, the ketones 10 may be converted to compounds of general formula 22 by treatment with an aromatic Grignard reagent or by the use of organolithium aromatic reagents in an inert solvent (such as anhydrous THF or Et₂O). Alcohols of general formula 22 may be converted to compounds of general formula 23 and 24 by the deprotection methods and N-alkylation by methods described in Scheme II.

Referring to schemes II, III and IV, the compounds prepared by these procedures are obtained as racemic mixtures and these maybe resolved into their constituent isomers by chiral chromatography techniques known to those skilled in the art of organic chemistry.

The present invention also includes within its scope solvates of the compounds of formula I and salts thereof, for example hydrates.

The effectiveness of the compounds of the invention in binding to specific receptor sites is determined by the following procedures.

### Membrane Preparation for Binding Assays

Cell pastes from large-scale production of HEK-293 cells expressing human α4β2, or α4β4 nAChRs are homogenised in 4 volumes of the appropriate buffer (50 mM Tris.HCl containing 150mM NaCl and 5mM KCl, pH 7.4). The homogenate is centrifuged twice (40,000 g, 10 minutes, 4 °C) and the pellets re-suspended in 4 volumes of Tris.HCl buffer after the first spin and 8 volumes after the second spin. The re-suspended homogenate is centrifuged (100 g, 10 minutes, 4 °C) and the supernatant kept and re-centrifuged (40,000 g, 20 minutes, 4 °C). The pellet is re-suspended in Tris.HCl buffer supplemented with 10 % w/v sucrose. The membrane preparation is stored in 1 ml aliquots at -80 °C until required. The protein concentration of the membrane preparation is determined using a BCA protein assay reagent kit.

### Nicotinic Receptor Radioligand Binding Scintillation Proximity Assay (SPA)

96-well SPA radioligand binding assays are performed in a final volume of 250 µl Tris-HCl buffer (50 mM Tris-HCl, 150 mM NaCl, 5 mM KCl, pH 7.4) with 1nM (α4β2) or 2nM (α4β4) [³H]-epibatidine (53 Ci/mmol; Amersham); 1 mg/well (α4β2) or 1.5 mg/well (α4β4) WGA-coated PVT SPA beads (Amersham) and 30 µg/well membrane protein for all 3 assay types. Non-specific binding (<10 % for all 3 assay types) is determined using 10 µM epibatidine.

Reactions are allowed to equilibrate for 2-4 hours at room temperature prior to reading on a Trilux Scintillation counter (Perkin Elmer). Data are analysed using a standard 4-parameter logistic equation (Multicalc, Perkin Elmer) to provide IC₅₀ values that are converted to Ki values using the Cheng-Prusoff equation (Cheng and Prusoff, 1973).

### [³H] Dopamine release assay from rat striatal slices

Release of [³H]dopamine from rat striatal slices is performed as follows. Male Lister-Hooded rats (250 - 350 g) are sacrificed by exposure to CO₂ followed by cervical dislocation. Striata from 2 rats is dissected and chopped 3 times at 150 µm using a McIlwain tissue chopper, each time rotating the tissue through 60°. Slices are dispersed in Krebs bicarbonate buffer (118 mM NaCl, 2.4 mM KCl, 2.4 mM CaCl₂.2H₂O, 1.2 mM KH₂PO_{4,} 1.2 mM MgSO₄.7H₂O, 25 mM NaHCO₃, 10 mM glucose, 1 mM ascorbic acid, gassed with 5% CO₂/95% O₂ for 1h, pH 7.4) including 10 µM pargyline, and incubated with [³H]dopamine (50 nM) for 30 minutes at 37°C. Following loading, slices are washed 4 times with Krebs buffer containing 1 µM nomifensine and 10 µM pargyline. After the final wash, slices are resuspended in Krebs buffer and a 100 µl aliquot placed in each well of a 96-well GF/C filter plate (Millipore). Buffer is removed to waste and 70 µl of buffer ± antagonist added to each well prior to incubation at 37°C for 5 minutes after which buffer is removed into a 96-well collection plate. Slices are then stimulated for 5 minutes with agonist (± antagonist; 70 µl/well), after which the stimulating buffer is removed into a 96-well collection plate.

Optiphase Supermix (Wallac) scintillation fluid (170 µl) is added to each well of the collection plates prior to plates being heat sealed and radioactivity quantified using a Wallac 1450 Microbeta 96-well plate counter (Wallac Oy, Turku, Finland, counting efficiency 25%). Radioactivity remaining in the slices is measured by digestion of the tissue in 0.2 ml Solvable (Packard Biosciences) for 1 h followed by addition of 0.5 ml isopropyl alcohol and 4.5 ml Hionic Fluor scintillation fluid (Packard Biosciences). Radioactivity was then quantified using a Wallac 1410 scintillation counter (Wallac Oy, Turku, Finland, counting efficiency 35%). Release of [³H]dopamine is expressed as a fraction of the total radioactivity contained within the slices at the time of stimulation.

The compounds exemplified in this application each have a binding affinity or potency at the α4β2 receptor (Kᵢ value) of less than 50nM. Moreover, each of the exemplified compounds also show a selectivity for the α4β2 receptor over the α4β4 receptor (i.e. Kᵢ α4β4/Kᵢ α4β2) of at least 2 fold. The compound according to Example 20 has the following biological profile:

| | α**4**β**2 Kᵢ** | α**3**β**4 Kᵢ** | **Ki** α**3**β**4/Kᵢ** α**4**β**2** | **Dopamine release EC₅₀** | **Efficacy Dopamine release %** |
|---|---|---|---|---|---|
| Example 20 | 5.37 | 479 | 89.2 | 135 | 41.7 |

The invention will now be illustrated with reference to the following non-limiting examples:

### Intermediate 1 Dimethyl 5-hydroxycyclohexane-1,3-dicarboxylate

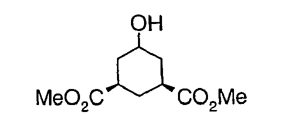

This intermediate is synthesized by hydrogenating dimethyl 5-hydroxyisophthalate utilizing the method of W J Gensler and P H Solomon (J. Org. Chem. 1973, 38(9), 1726-1731).

### Intermediate 2 Dimethyl 5-oxocyclohexane-1,3-dicarboxylate

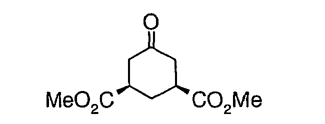

A method for synthesising this material is described by W J Gensler and P H Solomon (J. Org. Chem. 1973, 38(9), 1726-1731). Alternatively either of the following two methods can be used:

### Method 1:

A solution of oxalyl chloride (24mL, 0.276mol) in dry DCM (140mL) is added, with stirring, to a solution of DMSO (39.3mL, 0.553mol) in dry DCM (140mL), at -60°C. After 30 minutes a solution of dimethyl 5-hydroxycyclohexane-1,3-dicarboxylate (Intermediate 1,19.8g, 0.092mol) in DCM (100mL) is added at a rate that maintains the reaction temperature. The reaction mixture is stirred for 60 minutes, and then Et₃N (128mL, 0.92mol) is added at a rate that keeps the temperature below 0°C. The mixture is stirred for 120 minutes before ice and water are added to obtain two phases. These are separated; the organic phase is washed with 2M HCl, then brine; dried with MgSO₄; filtered; and evaporated *in vacuo.* The residue is triturated with Et₂O, and the solid filtered off and dried. Weight = 16.90g. Mass spectrum (m/z): 215(M+1);¹H-NMR (CDCl₃, 300MHz): δ 1.30-1.60 (2H, m), 2.15-2.5 (6H, m), 3.70 (6H, s) ppm; IR, 1722 cm⁻¹, 1700cm⁻¹_{.}

### Method 2:

5-Hydroxycyclohexane 1,3-dicarboxylic acid dimethyl ester (Intermediate 1, 100g, 463mmol) is dissolved in DCM (800mL) and cooled to -5°C under nitrogen. Water (50mL) is added, followed by potassium bromide (5.5g; 10mol%) and TEMPO^{®} (7.2g; 10ml%). To this resulting orange solution is added dropwise a slurry of NaHCO₃ (39.4g; leq) in sodium hypochlorite solution (5% free chlorine; 700mL), allowing the temperature to rise to 5°C. The reaction is armed to room temperature over 2h, then stirred with IPA (45mL) for 30 minutes. The mixture is transferred to a separating funnel and the aqueous layer extracted into DCM (2 x 200mL). The combined organic layers are washed with 1N NaOH solution (400mL), water (400mL) and brine (250mL). The solution is dried over MgSO₄, filtered and evaporated *in vacuo.* The resultant oil is triturated with Et₂O and the solid filtered off, washing with Et₂O (x2). Weight = 88.6g. Mass spectrum (m/z): 215(M+1); ¹H-NMR (CDCl₃, 300MHz): δ 1.30-1.60 (2H, m), 2.15-2.5 (6H, m), 3.70 (6H, s) ppm.

### Intermediate 3 Dimethyl 1,4-dioxaspiro[4,5]decane-7,9-dicarboxylate

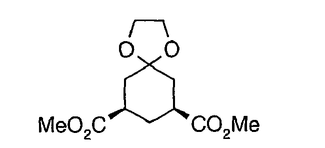

Dimethyl 5-oxocyclohexane-1,3-dicarboxylate (Intermediate 2, 2.14g, 0.01mol), ethylene glycol (0.56mL, 0.01mol) and PTSA (0.4g) are heated under reflux in toluene (75mL) for 4 hours, using a Dean and Stark apparatus to remove the water-toluene azeotrope. The cooled solution is washed with K₂CO₃ (aq), then brine, dried with MgSO₄, filtered, and evaporated *in vacuo.* The product is purified by trituration with Et₂O. Weight = 2.56g. Mass spectrum (m/z): 259(M+1); ¹H-NMR (CDCl₃, 300MHz): δ 1.45-1.68 (3H, m), 1.95-2.04 (2H, m), 2.20-2.30 (1H, m), 2.60-2.75 (2H, m), 3.70 (6H, s), 3.90-4.00 (4H, m) ppm.

### Intermediates 4 and 5 1,4-dioxaspiro[4,5]decane-7,9-dicarbaldehyde and 7-Benzylspiro[7-azabicyclo[3.3.1] nonane-3,2'-[1,3]dioxolanel

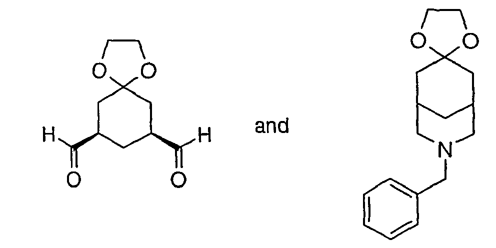

Dimethyl 1,4-dioxaspiro[4,5]decane-7,9-dicarboxylate (Intermediate 3, 38.5g, 0.1486mol) is dissolved in dry DCM (600mL) and cooled to -78°C, under a nitrogen atmosphere. A 1.5M solution of DIBAL^{®} in toluene (200mL, 0.3mol) is added at such a rate that the temperature does not exceed -60°C. On complete addition the reaction mixture is stirred at -78°C for 1 hours. The stirred reaction mixture is quenched with Rochelle's salt solution (1M) to give 2 layers. The resulting suspension is filtered. Tlc indicates successful formation of 1,4-dioxaspiro[4,5]decane-7,9-dicarbaldehyde (Example 4), as does the mass spectrum of (m/z): 199(M+1). The solvent is removed *in vacuo* and the resulting residue is purified by chromatography on SiO₂, eluting with Et₂O, to give a white solid. ¹H-NMR (CDCl₃, 300MHz): δ 1.20-1.30 (1H, m), 1.40-1.50 (2H, m), 2.00-2.10 (2H, m), 2.28-2.35 (1H, d), 2.60-2.70 (2H, m), 3.90-4.02 (4H, m), 9.65 (2H, s) ppm.

1,4-Dioxaspiro[4,5]decane-7,9-dicarbaldehyde (Intermediate 4) is dissolved in DCM (600 mL) and benzylamine (16.2mL, 0.1486mol) is added at ambient temperature. After stirring the resulting solution for 15 minutes sodium triacetoxyborohydride (94.48g, 0.4458mol) is added. The reaction mixture is then stirred for 24 hours. 2M NaOH (450mL, 0.9mol) is added to give a pH between 9 and 10. The mixture is shaken, then the phases separated. The organic phase is washed with brine, dried with MgSO₄, filtered, and evaporated *in vacuo.* Weight of 7-benzylspiro[7-azabicyclo[3.3.1]]nonane-3,2'-[1,3]dioxolane] (Example 5) = ∼41g. Mass spectrum (m/z): 274(M+1); ¹H-NMR (CDCl₃, 300MHz): δ 1.30-1.40 (1H, d), 1.75-1.90 (5H, m), 1.98-2.10 (2H, s), 2.40-2.50 (2H, d), 2.80-2.90 (2H, d), 3.70-3.75 (2H, s), 3.80-3.90 (4H, m), 7.15-7.30 (3H, m), 7.30-7.45 (2H, m) ppm.

### Intermediate 6 Spiro[7-azabicyclo[3.3.1]nonane-3,2'-[1,3]dioxolane]

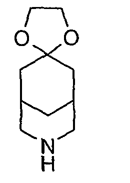

A solution of 7-benzylspiro[7-azabicyclo[3.3.1]nonane-3,2'-[1,3]dioxolane] (Intermediate 5, 18.0g, 0.066mol) in EtOH (50mL) is added, in a steady stream, to a stirred suspension of 20% Pd(OH)₂ on carbon (9g) in EtOH (100mL), at ambient temperature, under a nitrogen atmosphere. Cyclohexene (45.0mL, 0.444mol) is added to the reaction mixture, and this is heated under reflux for 1 hour 45 minutes. The cooled solution is filtered off through Celite, and evaporated *in vacuo* to give an orange-brown oil. Weight = 14.12g. Mass spectrum (m/z): 184(M+1); ¹H-NMR (CDCl₃, 300MHz): δ 1.62-1.85 (4H, mixture of multiplets), 1.92-1.98 (4H, s), 2.90-2.92 (4H, s), 3.92-3.98 (2H, m), 24.00-4.04 (2H, m) ppm. NH proton is not apparent.

### Intermediate 7 3-Benzyl-3-azabicyclo[3.3.1]nonan-7-one

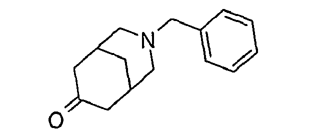

7-Benzylspiro[7-azabicyclo[3.3.1]nonane-3,2'-[1,3]dioxolane] (Intermediate 5, 18.62g, 0.0681mol) is dissolved in 2M HCl (170mL, 0.34mol) and heated under reflux for 24 hours. Solid K₂CO₃ (24g, 0.174mol) is added in portions to the cooled solution. The suspension is extracted into CHCl₃, and the organic extract is washed with brine, dried with MgSO₄, filtered, and evaporated *in vacuo.* Weight = 9.0g. Mass spectrum (m/z): 230(M+1); m.p. = 89°C (when purified by column chromatography); ¹H-NMR (CDCl₃, 300MHz): δ 1.68-1.78 (1H, d), 1.80-1.90 (1H, d), 2.18-2.28 (4H, m), 2.36-2.40 (4H, m), 2.70-2.75 (2H, d), 3.45-3.50 (2H, s), 7.18-7.22 (3H, m), 7.27-7.35 (2H, m) ppm.

### Intermediate 8 Benzyl 7-oxo-3-azabicyclo[3.3.1]nonane-3-carboxylate

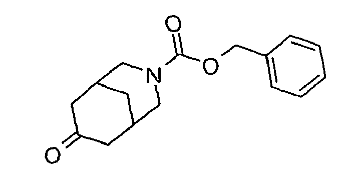

Et₃N (3.0mL, 0.0224mol) is added to a stirred solution of spiro[7-azabicyclo[3.3.1]nonane-3,2'-[1,3]dioxolane] (Intermediate 6, 3.87g, 0.0187mol) in CHCl₃ (50mL) at 0°C. Benzyl chloroformate (2.68mL, 0.0187mol) is added in a steady stream to the reaction mixture, which is then stirred at 0°C for 1 hour. The reaction mixture is allowed to warm to ambient temperature and stirred for 24 hours. The solution is evaporated *in vacuo*, the residue is dissolved in EtOAc (Et₃N.HCl is deposited). Enough 1M NaOH is added to give two clear phases, and to render the aqueous phase basic. The two phases are shaken and separated. The organic phase is washed with 1M NaOH, and then brine, dried with MgSO₄ filtered and evaporated *in vacuo.* The residue is purified by chromatography on SiO₂ eluting with cyclohexane:EtOAc (20-75%). Weight of benzyl 7H-spiro[7-azabicyclo[3.3.1]nonane-3,2'-[1,3]dioxolane]-7-carboxylate intermediate (a golden brown oil) = 5.59g. Mass spectrum (m/z): 318(M+1).

This intermediate (5.59g, 0.0176mol) is dissolved in THF (160mL) and cooled to 0°C and, 2M HCl (24.0 mL, 0.024mol) is added in one portion with stirring. The resulting solution is warmed to ambient temperature, and stirred for 2 hours. The reaction mixture is concentrated *in vacuo,* and the residual oil is diluted with EtOAc and washed with brine. The organic phase is dried with MgSO₄, filtered and evaporated *in vacuo.* Weight of colourless clear oil (which crystallizes on standing) = 2.6g. Mass spectrum (m/z): 274(M+1); ¹H-NMR (CDCl₃, 300MHz): δ 1.85-1.92 (1H, d), 1.93-2.02 (1H, d), 2.25-2.33 (2H, broad s), 2.40-2.50 (4H, s), 2.88-3.02 (2H, broad s), 4.02-4.30 (2H, m), 5.03-5.18 (2H, s), 7.25-7.40 (5H, m) ppm.

### Intermediate 9 tert-Butyl 7-oxo-3-azabicyclo[3.3.1]nonane-3-carboxylate

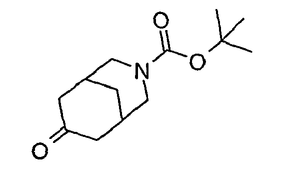

Et₃N (9.25mL, 0.066mol), is added, with stirring, to a solution of spiro[7-azabicyclo[3.3.1]]nonane-3,2'-[1,3]dioxolane (Intermediate 6, 14.12g, 0.066mol) in DCM (100mL) at 0°C. A solution of di-*tert*-butyl dicarbonate (14.83g, 0.068mol) in DCM (70mL) is added dropwise. On completion of the addition the reaction mixture is stirred for 1 hour at 0°C, then warmed to ambient temperature and stirred for 24 hours. The reaction mixture is evaporated *in vacuo,* the residue is dissolved in EtOAc and this solution washed with water, 1M HCl, and then brine. The organic solution is dried with MgSO₄, filtered, and evaporated *in vacuo.* The residual oil is purified by chromatography on SiO₂, eluting with *iso*-hexane:EtOAc (20-77%). Weight of *tert*-butyl 7H-spiro[7-azabicyclo[3.3.1]nonane-3,2'-[1,3]dioxolane]-7-carboxylate intermediate (colourless clear oil) = 13.6g. Mass spectrum (m/z): 306(M+23)/589(2M+23).

PTSA (0.068g) is added to a solution of this intermediate (13.6g, 0.0481mol) in acetone (80mL), and stirred at ambient temperature for 24 hours. The reaction mixture is evaporated *in vacuo,* the residue is dissolved in EtOAc and washed with saturated Na₂CO₃ solution, and then brine. The organic extract is dried with MgSO₄ filtered and evaporated to give a pale yellow oil that crystallizes on standing. Weight = 11.0g. Mass spectrum (m/z): 262(M+23); ¹H-NMR (CDCl₃. 300MHz): δ 1.43 (9H, s), 1.82-1.88 (1H, d), 1.92-1.98 (1H, d), 2.28-2.34 (2H, broad s), 2.40-2.45 (4H, broad s), 2.80-2.95 (2H, broad s), 3.92-4.20 (2H, m) ppm.

### Intermediate 10 tert-Butyl 7-{[(trifluoromethyl)sulfonyl]oxy}-3-azabicyclo[3.3.1]non-6-ene-3-carboxylate

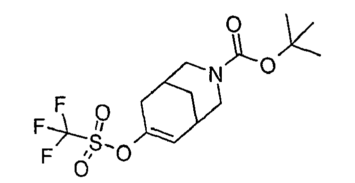

A solution of *tert*-Butyl 7-oxo-3-azabicyclo[3.3.1]nonane-3-carboxylate (Intermediate 9, 59.2g, 0.250mol) in dry THF (600mL) is added with stirring, under a nitrogen atmosphere to a solution of LiHMDS (300mL, 0.3mol) in dry THF (600mL), at -78°C, at a rate that maintains this reaction temperature. After a further 30min a solution of *N*-phenyl trifluoromethane sulfonimide (98.3g, 0.275mol) in dry THF (600mL) is added dropwise over 1 hour and stirred for a further 30 min at this temperature and then allowed to warm to 10°C over 2 hours. The reaction mixture is poured onto brine (750mL) and water (250mL) is added. The product is extracted into Et₂O (2x1L). The combined organic extracts are washed with water (3x1L) and brine (2x1L), dried over anh MgSO₄, filtered and the filtrate evaporated *in vacuo.* The residue is purified by chromatography on a silica pad eluting with iso-hexane/Et₂O (0-5%) and then (5-35%) to obtain pure product. Weight = 60.6g. ¹H-NMR (CDCl₃, 300MHz): δ 1.42 (9H, s), 1.62 - 1.89 (2H, m), 2.07 - 2.45 (2H, m), 2.47 - 2.71 (2H, m), 2.87 (2H, m), 3.81 - 4.34 (2H, m), 5.75 (1H, s) ppm.

### Intermediate 11

### tert-Butyl 7-(4,4,5,5-tetramethyl-1,3,2-dioxaborolan-2yl)-3-azabicyclo[3.3.1]non-6-ene-3-carboxylate

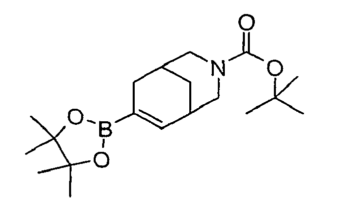

*tert*-Butyl 7-{[(trifluoromethyl)sulfonyl]oxy}-3-azabicyclo[3.3.1]non-6-ene-3-carboxylate (Intermediate 10, 20g, 0.054mol), bis(pinacol)diborane (15g, 0.059mol), potassium acetate (15.9g, 0.162mol), 1,1'-bis(diphenylphosphino)ferrocenedichloro palladium (II) (2.2g, 0.0027mol) and 1,1'-bis(diphenylphosphino)ferrocene (1.5g, 0.0027mol) in 1,4-dioxane (350mL) are degassed for 30 min, then heated at 80°C overnight. The reaction mixture is cooled to ambient temperature and is poured onto brine (250mL). The resulting solution is extracted with EtOAc (2x250mL). The combined organic extracts were dried over anh MgSO₄, filtered and the filtrate concentrated *in vacuo.* The resulting oil is absorbed onto SiO₂ and loaded onto the top of a SiO₂ pad and eluted with *iso-*hexane/EtOAc (10 to 50% gradient) to give a yellow oil which solidifies on standing. Weight = 10.9g. Mass spectrum (m/z): 372 (M+Na); ¹H-NMR (CDCl₃, 300MHz): δ 1.26 (12H, s), 1.43 (9H, s), 1.64 - 1.77 (2H, m), 1.88 - 2.02 (1H, m), 2.12 - 2.47 (2H, m), 2.65 - 3.04 (2H, m), 3.54 - 4.31 (3H, m), 6.55 (1H, dd, *J*=19.4, 5.5 Hz) ppm.

### Intermediate 12

### tert-Butyl 7-(3-fluorophenyl)-3-azabicyclo[3.3.1]non-6-ene-3-carboxylate

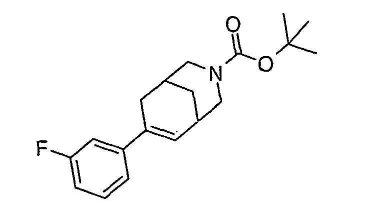

To *tert*-butyl 7-{[(trifluoromethyl)sulfonyl]oxy}-3-azabicyclo[3.3.1]non-6-ene-3-carboxylate (Intermediate 10, 0.25g, 0.00067mol), 3-fluorobenzene boronic acid (0.14g, 0.001mol), lithium chloride (0.09g, 0.0021mol) and Na₂CO₃ (1mL, 2M) in ethylene glycol dimethyl ether (2mL) is added tetrakis(triphenylphosphine) palladium (0) (0.039g, 0.00034mol). The mixture is heated to reflux for 24 hours. The reaction mixture is allowed to cool to ambient temperature then diluted with DCM (10mL), washed with aqueous Na₂CO₃ solution (2M, 5mL) and a few drops of NH₄OH solution. The organic extract is dried over Na₂SO₄, filtered, and evaporated *in vacuo.* The residue is purified by chromatography on SiO₂ eluting with *iso*-hexane and EtOAc to give a clear oil. Weight = 0.05g. Mass spectrum (m/z): 318 (M+1); ¹H-NMR (CDCl₃, 300MHz): δ 1.25 (9H, s), 1.65 - 1.93 (2H, m), 2.14 (1H, s), 2.25 - 2.64 (2H, m), 2.66 - 3.10 (3H, m), 3.89 - 4.35 (2H, m), 6.12 (1H, dd, *J*=25.2, 6.2 Hz), 6.89 (1H, dt), 7.07 (1H, d, *J*=10.9 Hz), 7.12 - 7.28 (2H, m) ppm.

### Intermediate 13

### tert-Butyl 7-(3,4-difluorophenyl)-3-azabicyclo[3.3.1]non-6-ene-3-carboxylate

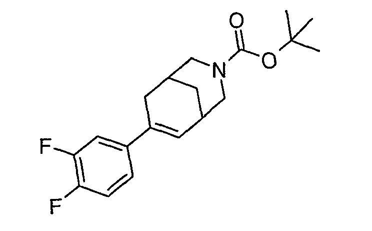

To *tert*-butyl 7-{[(trifluoromethyl)sulfonyl]oxy}-3-azabicyclo[3.3.1]non-6-ene-3-carboxylate (Intermediate 10, 0.086g, 0.000232mol), 3,4-difluorophenyl boronic acid (0.072g, 0.000464mol), and Na₂CO₃ (0.049g, 0.000464mol) in THF (10mL) and water (5mL) is added bis(triphenylphosphine)palladium (II) chloride (0.016g, 0.000023mol). The mixture is heated under reflux for 24 hours. The black reaction mixture is allowed to cool to ambient temperature then poured onto brine (50mL) and extracted with Et₂O(2 x 25mL). The organic extract is further washed with 2N NaOH solution (20mL), dried over MgSO₄ and concentrated *in vacuo* to a pale brown oil. Weight = 0.090g. ¹H-NMR (CDCl₃, 300MHz): δ 1.19 (9H, s), 1.60 - 1.88 (2H, m), 1.95 - 2.30 (2H, m), 2.33 - 2.54 (2H, m), 2.54 - 3.06 (2H, m), 3.70 - 4.37 (2H, m), 6.02 (1H, s), 6.89 - 7.05 (1H, m), 7.31 - 7.50 (1H, m), 7.52 - 7.69 (1H, m) ppm.

### Intermediate 14 tert-Butyl 7-(3-bromo-5-fluorophenyl)-3-azabicyclo[3.3.1]non-6-ene-3-carboxylate

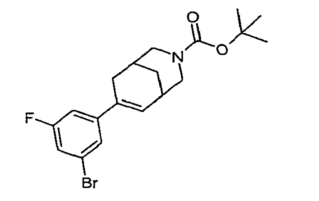

To *tert*-Butyl 7-(4,4,5,5-tetramethyl-1,3,2-dioxaborolan-2yl)-3-azabicyclo[3.3.1]non-6-ene-3-carboxylate (Intermediate 11, 0.85g, 0.00234mol), dibromofluorobenzene (1.837mL, 0.0146mol) and Na₂CO₃ (0.25g, 0.02434mol) in THF (70mL) and water (35mL) is added bis(triphenylphosphine)palladium(II) chloride (0.171g, 0.000243mol). The mixture is heated to 80°C for 4 hr. The reaction mixture is cooled to ambient temperature, is poured into 2M NaOH (100mL) and is extracted with EtOAc (2x60mL). The organic extract is dried over MgSO₄, filtered, and evaporated *in vacuo.* The residue is purified by chromatography on SiO₂ eluting with *iso*-hexane and EtOAc to give a yellow oil. Weight = 0.38g. 1H NMR (CDCl₃, 300 MHz) δ ppm 1.21 (9 H, s), 1.23 - 1.26 (1 H, m), 1.76 - 1.85 (1 H, m), 2.07 - 2.20 (1 H, m, *J*=7.2 Hz), 2.45 - 2.59 (1 H, m), 2.76 - 3.11 (2 H, m), 3.89 (1 H, d, *J*=13.0 Hz), 4.05 (1 H, d, *J*=13.2 Hz), 4.13 (1 H, d, *J*=13.4 Hz), 4.33 (1 H, d, *J*=13.8 Hz), 6.12 (1 H, dd, *J*=20.2, 6.4 Hz), 6.94 - 7.04 (1 H, m), 7.05 - 7.13 (1 H, m), 7.28 - 7.32 (1 H, m).

### Intermediate 15 3-Bromo-5-pyridin-4-ylbenzonitrile

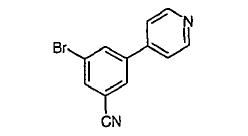

To dibromobenzonitrile (5g, 0.019mol), 4-pyridyl boronate ester (5g, 0.024mol), Na₂CO₃ (0.25g, 0.02434mol) in THF (50mL) and water (25mL) is added bis(triphenylphosphine)palladium(II) chloride (1.43g, 0.02mol). The mixture is heated to 80°C for 2 hr. The reaction mixture is cooled to ambient temperature, is poured into a saturated solution of sodium chloride (80mL) and is extracted with EtOAc (2x50mL). The organic extract is dried over MgSO₄, filtered, and evaporated *in vacuo.* The residue is purified by chromatography on SiO₂, eluting with *iso*-hexane and EtOAc to give a white solid. MeOH was added and the resultant whilte precipitate filtered and dried. Weight = 1.9g. 1H NMR (300 MHz, *CHLOROFORM-d*) δ ppm 7.47 (2 H, dd, *J*=4.4,1.6 Hz), 7.85 (2 H, dt, *J*=5.6, 1.5 Hz), 8.00 (1 H, t, *J=*1.7 Hz), 8.74 (2 H, dd, *J*=4.5, 1.5 Hz)

### Intermediate 16 1-Fluoro-3-methoxy-5-[(trifluoromethyl)sulfonyloxy]benzene

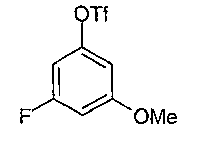

To 5-methoxybenzene-1,3-diol [Org. Synth. 53, 1973, 90-93] (4.71g, 0.032mol) in dry THF (200mL) under an atmosphere of nitrogen is added NaH (60% dispersion in mineral oil, 0.038mol) over 15 minutes resulting in effervescence and a peach coloured solution. 1,1,1-trifluoro-N-phenyl-N-[(trifluoromethyl)sulphonyl]methanesulfonimide (13.73g, 0.038mol) is added over 2 minutes resulting in effervescence and a yellow suspension. Stirring is continued at ambient temperature for 20h. The reaction mixture is poured into a saturated solution of NaCl (200mL) and extracted with Et₂O (2x200mL). The organic extracts are dried over MgSO₄, filtered, and evaporated *in vacuo.* The residue is purified by chromatography on SiO₂, eluting with *iso*-hexane and EtOAc to give a yellow oil. Weight = 6.3g. 1H NMR (CDCl₃, 300 MHz) δ ppm 3.80 - 3.84 (3 H, m), 6.61 - 6.68 (2 H, m, *J*=11.5, 4.2, 2.4, 2.1 Hz), 7.49 - 7.61 (1 H, m).

Bromo-3-fluoro-5-(NMe-1,2,4-triazole) and bromo-3-fluoro-5-([1,2,4-oxadiazole]) are prepared according to J. Org Chem. 44(23), 1979, 4160. Bromo-3-fluoro-5-(NMe-2-pyrrole) is prepared according to Tet. Lett. 27(37), 1986, 4407. Bromo-3-fluoro-5-(2-thiazole) is prepared according to WO 03/072547. 1-(3-Bromo-5-fluorophenyl)-ethanone is prepared according to EP 86-103580 19860317. 5-(3-Bromo-5-fluorophenyl)-3-methylisoxazole prepared according to Heteroatom Chemistry 15(1), 2004, 85-91.

Other substituted aryl intermediates are synthesised in a similar manner to Intermediate 14 from the respective bromo or trifluoromethylsulphonyl aryls which maybe prepared by methods described herein or by known literature procedures. In some cases the intermediates are taken on without purification through to the deprotection step.

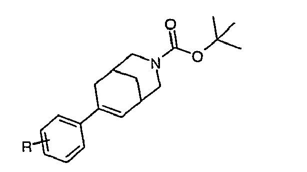

| **Interm^{ediate}** | **R** | **Data** |
|---|---|---|
| **17** | 3,5-diF | ¹H-NMR (CDCl₃, 300MHz): δ 1.26 (9H, s), 1.67 - 1.93 (2H, m), 2.00-3.11 (6H, m), 3.83 - 4.43 (2H, m), 6.00 - 6.27 (1H, m), 6.76 - 6.97 (2H, m), 7.49 - 7.75 (1H, m) ppm. |
| **18** | 3,4,5-triF | ¹H-NMR (CDCl₃, 300MHz): δ 1.20 (9H, s), 1.63 - 1.82 (2H, m), 1.96 - 3.03 (6H, m), 3.71 - 4.33 (2H, m), 5.89 - 6.10 (1H, m), 6.80 - 6.98 (2H, m) ppm. |
| **19** | 2-F | ¹H-NMR (CDCl₃, 300MHz): δ 1.34 (9H, s), 1.68 - 1.97 (2H, m), 1.98 - 3.08 (6H, m), 3.82 - 4.39 (2H, m), 5.83 - 6.01 (1H, m), 6.92 - 7.10 (2H, m), 7.11 - 7.26 (2H, m) ppm. |
| **20** | 3-CN | ¹H-NMR (CDCl₃, 300MHz): δ 1.25 (9H, s), 2.00 - 2.42 (2H, m), 2.49 - 3.14 (4H, m), 3.66 - 3.79 (2H, m), 3.84 - 4.42 (2H, m), 6.06 - 6.28 (1H, m), 7.32 - 7.56 (4H, m) ppm. |
| **21** | 3-F, 5-CF₃ | Taken on without purification. Analysis on final compound Example 8 |
| **22** | 3-F, 4-F, 5-OMe | Taken on without purification. Analysis on final compound Example 40 |
| **23** | 3-F, 5-OMe | 1H NMR (CDCl₃,300 MHz) δ ppm 1.22 (1 H, d, *J*=2.3 Hz), 1.22 - 1.33 (9 H, m), 1.40 (1 H, d, *J*=5.3 Hz), 1.69 - 1.84 (1 H, m), 2.09 - 2.18 (1 H, m, *J*=3.6 Hz), 2.29 - 2.39 (1 H, m, *J*=0.8 Hz), 2.45 - 2.58 (1 H, m), 2.84 - 3.00 (1 H, m), 3.78 - 3.82 (3 H, m), 3.82 - 3.95 (1 H, m), 4.00 - 4.12 (1 H, m), 4.25 - 4.41 (1 H, m, *J*=14.1 Hz), 6.10 (1 H, d, *J*=23.7 Hz), 6.47 (1 H, dt, *J*=10.5, 2.3 Hz), 6.66 - 6.72 (2 H, m). |
| **24** | 3-F, 5-(NMe-1,2,4-triazole) | 1H NMR (CDCl₃, 300 MHz) δ ppm 1.23 - 1.25 (9 H, m), 1.26 - 1.27 (1 H, m), 1.64 (1 H, s), 1.81 (1 H, s), 1.86 - 1.99 (2 H, m), 2.42 (1 H, s), 2.61 (1 H, s), 2.88 (1 H, s), 3.97 - 4.02 (3 H, m), 4.11 (1 H, ddd, *J*=14.6, 7.6, 7.4 Hz), 4.27 - 4.40 (1 H, m), 6.13 - 6.29 (1 H, m), 7.20 - 7.27 (2 H, m), 7.48 (1 H, s), 7.90 - 7.95 (1 H, m). |
| **25** | 3-F, 5-(NMe-2-pyrrole) | 1H NMR (CDCl₃, 300 MHz) δ ppm 1.22 (1 H, d, *J*=2.3 Hz), 1.26 (9 H, s), 1.36 - 1.43 (1 H, m), 1.65 (1 H, s), 2.10 - 2.21 (1 H, m), 2.47 - 2.63 (1 H, m), 2.70 - 2.83 (1 H, m), 2.83 - 2.98 (1 H, m), 3.62 - 3.68 (3 H, m), 3.83 - 3.95 (1 H, m, *J*=8.7 Hz), 3.97 - 4.18 (1 H, m), 4.26 - 4.39 (1 H, m, *J*=12.2 Hz), 6.15 - 6.24 (2 H, m), 6.71 (1 H, t, *J*=2.2 Hz), 6.91 - 7.04 (2 H, m), 7.19 (1 H, s). |
| **26** | 3-F, 5-(2-thiazole) | 1H NMR (CDCl₃, 300 MHz) δ ppm 1.23 (1 H, d, *J*=4.1 Hz), 1.27 (9 H, s), 1.40 (1 H, d, *J*=11.9 Hz), 1.81 (2 H, s), 2.17 (1 H, s), 2.56 - 2.67 (1 H, m), 2.82 - 2.95 (1 H, m, *J*=14.9 Hz), 2.97 - 3.07 (1 H, m), 3.87 - 3.98 (1 H, m), 4.04 - 4.19 (1 H, m), 6.17 - 6.28 (1 H, m), 7.14 (1 H, d, *J*=10.2 Hz), 7.32 - 7.39 (1 H, m), 7.54 (1 H, ddd, *J*=9.1, 2.4, 1.5 Hz), 7.71 - 7.76 (1 H, m), 7.87 (1 H, d, *J*=3.2 Hz). |
| **27** | 3-F, 5-(5-[1,2,4-oxadiazole]) | 1H NMR (CDCl_{3,} 300 MHz) δ ppm 0.83 (1 H, d, *J*=1.9 Hz), 1.18 - 1.31 (9 H, m), 1.34 - 1.44 (2 H, m), 1.83 (1H, s), 2.19 (1 H, s), 2.51 - 2.65 (1 H, m), 2.89 (1 H, s), 3.84 - 3.98 (1 H, m), 4.11 (1 H, ddd, *J*=13.9, 6.9, 6.7 Hz), 4.35 (1 H, d, *J*=13.4 Hz), 6.18 - 6.37 (1 H, m), 7.29 - 7.39 (1 H, m), 7.63 - 7.74 (1 H, m), 7.92 - 8.04 (1 H, m), 8.49 (1 H, s). |
| **28** | 3-F, 5-acetyl | 1H NMR (CDCl₃, 300 MHz) δ ppm 1.17 - 1.30 (9 H, m), 1.39 (1 H, s), 1.60 (2 H, s), 2.18 (1 H, d, *J*=6.2 Hz), 2.55 - 2.62 (3 H, m), 2.78 (1 H, d), 2.84 - 2.97 (1 H, m), 2.97 - 3.09 (1 H, m), 3.86 - 3.98 (1 H, m), 4.00 - 4.22 (1 H, m), 4.26 - 4.42 (1 H, m), 6.11 - 6.27 (1 H, m), 7.29 (1 H, s), 7.40 - 7.53 (1 H, m), 7.75 (1 H, s). |
| **29** | 3-F, 5-(3-methylisoxazol-5-yl) | 1H NMR (300 MHz, *CHLOROFORM-d*) δ ppm 1.26 (9 H, s), 1.82 (2 H, d, *J*=2.8 Hz), 2.18 (1 H, t, *J*=11.2 Hz), 2.33 - 2.37 (3 H, m), 2.53 - 2.63 (2 H, m), 2.80 (1 H, s), 2.88 (1 H, s), 3.06 (1 H, s), 4.12 (2 H, q, *J*=7.0 Hz), 6.18 (1 H, d, *J*=20.9 Hz), 6.38 (1 H, s), 7.13 (1 H, ddd, *J*=10.2, 2.4,1.6 Hz), 7.29 (1 H, dt, *J*=8.1, 1.5 Hz), 7.54 (1 H, s). |
| **30** | 3-F, 5-ethoxy | 1H NMR (300 MHz, CHLOROFORM-*d*) δ ppm 1.26 (s, 9 H) 1.39 (s, 3 H) 1.80 (s, 2 H) 2.14 (s, 1 H) 2.51 (s, 2 H) 2.60 - 3.10 (m, 2 H) 3.83 - 3.93 (m, 1 H) 4.04 (s, 2 H) 4.03 - 4.18 (m, 1 H) 4.24 - 4.37 (m, 1 H) 6.09 (s, 1 H) 6.46 (s, 1 H) 6.69 (s, 1 H) 7.26 (s, 1 H). |
| **31** | 3-CN, 5-pyridin-4-yl | 1H NMR (300 MHz, CHLOROFORM-*d*) δ ppm 1.21 - 1.28 (m, 9 H) 1.65 (s, 2 H) 1.84 (s, 2 H) 2.20 (s, 1 H) 2.63 (s, 2 H) 2.90 (s, 1 H) 3.72 - 4.52 (m, 2 H) 6.27 (s, 1 H) 7.46 - 7.50 (m, 2 H) 7.61 - 7.75 (m, 3 H) 7.79 - 7.87 (m, 1 H) 8.70 - 8.73 (m, 1 H). |
| **32** | 3-F, 5-phenyl | Taken on without purification. Analysis on final compound Example 41 |
| **33** | 3-F, 5-(3-furan) | 1H NMR (CDCl3, 300 MHz) □ ppm 1.27 (9 H, s), 1.57 (1 H, s), 1.80 (2 H, s), 2.17 (1 H, s), 2.50 (1 H, s), 2.60 (1 H, s), 2.90 (1 H, d, J=18.1 Hz), 3.81 - 3.96 (1 H, m), 4.14 (1 H, d, J=12.4 Hz), 4.32 (1 H, s), 6.08 - 6.20 (1 H, m), 6.67 (1 H, dd, J=1.8, 0.8 Hz), 6.97 (1 H, d, J=10.4 Hz), 7.02 (1 H, dt, J=9.5, 1.9 Hz), 7.26 (1 H, s), 7.47 (1 H, t, J=1.7 Hz), 7.71 - 7.74 (1 H, m). |

### Intermediate 34 tert-Butyl 7-[3-fluoro-5-(1H-imidazol-1-yl)phenyl]-3-azabicyclo[3.3.1]non-6-ene-3-carboxylate

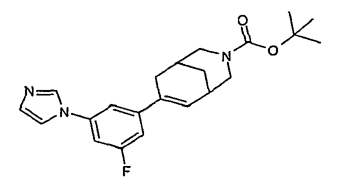

To *tert*-butyl 7-(3-bromo-5-fluorophenyl)-3-azabicyclo[3.3.1]non-6-ene-3-carboxylate (Intermediate 14, 0.13g, 0.000328mol) in dry DMF (2mL) is added imidazole (0.045g, 0.000656mol), K₂CO₃ (0.091g, 0.000656mol) and CuI (0.006g, 0.000033mol). The mixture is heated to 150°C for 24 hr. The reaction mixture is allowed to cool to ambient temperature and is poured into a saturated solution of NH₄Cl (50mL) and extracted with Et₂O (2x50mL). The aqueous phase was re-extracted with DCM (2x50mL) and combined organic extracts are dried over MgSO₄, filtered, and evaporated *in vacuo.* The residue is a brown oil of low purity. Weight = 0.163g. Mass spectrum (m/z): 384(M+1).

Other substituted aryl intermediates are synthesized in a similar manner to Intermediate 34 from the respective bromo aryl.

| | | |
|---|---|---|
| **Intermedate 35** | 3-F, 5-phenoxy | Mass spectrum (m/z): 432.2 (M+Na) |

### Intermediate 36

### tert-Butyl 7-(3-fluoro-5-pyrimidin-2-ylphenyl)-3-azabicyclo[3.3.1]non-6-ene-3-carboxylate

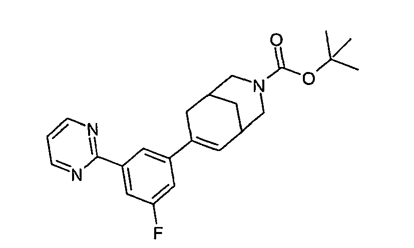

Under an atmosphere of nitrogen, nBuLi (2.5M, 0.45mL, 00.114mol) is added to dry THF (5mL) cooled to -78°C by a solid CO₂-acetone mixture. *tert-Butyl* 7-(3-bromo-5-fluorophenyl)-3-azabicyclo[3.3.1]non-6-ene-3-carboxylate (Intermediate 14, 0.3g, 0.000757mmol) is added as a solution in dry THF (2mL) over 5 minutes maintaining the temperature below -70°C. The reaction mixture is stirred at -78°C for 8 minutes. A solution of ZnCl₂ in dry THF (0.5M, 2.88mL, 0.00144mol) is added dropwise over 10 minutes, maintaining the temperature below -70°C, giving a bright yellow solution. Stirring is continued at -78°C for 1h. A solution of 2-bromo pyrimidine (0.144g, 0.000908mol) and tetrakis(triphenylphosphine) palladium (0) (0.0889g, 0.000076mol) in dry DMF is added dropwise over 5 minutes. The reaction is warmed to ambient temperature over 20 minutes and is heated at 80°C for 5h. The reaction mixture is cooled to ambient temperature and is poured into a saturated solution of NaCl (30mL) and extracted with EtOAc (2x20mL). The organic extracts are dried over MgSO₄, filtered, and evaporated *in vacuo.* The residue is purified by chromatography on SiO₂, eluting with *iso*-hexane and EtOAc to give a yellow oil. Weight = 0.044g. 1H NMR (CDCl₃, 300 MHz) δ ppm 1.11 - 1.22 (9 H, m), 1.66 - 1.82 (2 H, m), 2.02 - 2.13 (1 H, m), 2.39 - 2.54 (2 H, m), 2.64 - 2.80 (1 H, m), 2.80 - 2.94 (1 H, m), 3.74 - 3.90 (1 H, m), 3.94 - 4.10 (1 H, m), 4.26 (1 H, d, *J*=13.8 Hz), 6.08 (1 H, d, *J*=3.2 Hz), 6.78 - 6.86 (1 H, m), 6.97 - 7.08 (1 H, m), 7.10 (1 H, s), 7.12 - 7.26 (3 H, m).

### Intermediate 37

### tert-Butyl 7-(3-fluoro-5-pyridin-4-ylphenyl)-3-azabicyclo[3.3.1]non-6-ene-3-carboxylate

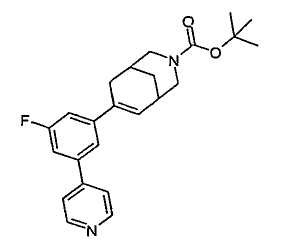

To *tert*-butyl 7-(3-bromo-5-fluorophenyl)-3-azabicyclo[3.3.1]non-6-ene-3-carboxylate Intermediate 14, 0.0634g, 0.00016mol), 4-pyridyl boronic acid (0.039g, 0.0003mol) and Na₂CO₃ (0.034g, 0.00032mol) in THF (5mL) and water (2.5mL) is added bis(triphenylphosphine)palladium(II) chloride (0.011g, 0.00002mol). The mixture is heated to 80°C for 4 hr. The reaction mixture is cooled to ambient temperature, is poured into 2M NaOH (30mL) and is extracted with EtOAc (2x20mL). The organic extract is dried over MgSO₄ filtered, and evaporated *in vacuo.* Weight = 0.071g. The material is used crude in the next step.

### Intermediate 38

### tert-Butyl 7-(3,5-difluorophenyl)-3-azabicyclof3.3.llnonane-3-carboxylate

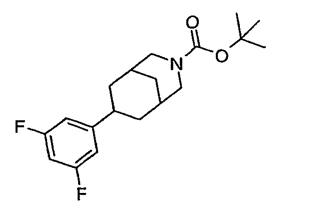

*tert*-Butyl 7-(3,5-difluorophenyl)-3-azabicyclo[3.3.1]non-6-ene-3-carboxylate (0.091g, 0.00023mol) is dissolved in MeOH (15mL) in a Parr flask and 10% Pd-C (0.035g) is added. The reaction is subjected to hydrogenation under 65psi of H₂ with vigorous stirring for 3 days. The reaction mixture is filtered and washed with MeOH (50mL). The filtrate is concentrated *in vacuo* to an oil. Weight = 0.10g. Mass spectrum (m/z): 260 (M+Na); ¹H-NMR (CDCl₃, 300MHz): δ 1.43 (9H, s), 1.47 (3H, m), 1.83 - 2.11 (5H, m), 2.49 - 2.83 (3H, m), 3.75 - 4.08 (2H, m), 6.48 - 6.57 (1H, m), 6.60 - 6.68 (2H, m) ppm.

### Example 1 7-(3-Fluorophenyl)-3-azabicyclo[3.3.1]non-6-ene (L) (+) tartrate

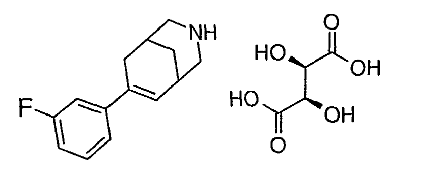

*tert*-Butyl 7-(3-fluorophenyl)-3-azabicyclo[3.3.1]non-6-ene-3-carboxylate (Intermediate 12, 0.005g, 0.00016mol) is dissolved in dry DCM, and TFA (0.5mL) is added at ambient temperature. The reaction mixture is stirred for 2 hours 30 minutes. The brown mixture is evaporated *in vacuo* and absorbed onto a SCX-2 cartridge^{®} (5g). The ion exchange cartridge is washed with MeOH, and the product then eluted with MeOH / NH₃ (7N). The eluate is concentrated *in vacuo* to give a clear oil. Weight = 0.03g. This material is dissolved in a minimum amount of MeOH (1mL) and to this solution is added a solution of (L)-(+)-tartaric acid (0.021g, 0.00014mol) in MeOH (1mL). The mixture is concentrated *in vacuo* to a white solid. Weight = 0.0405g. Mass spectrum (m/z): 218 (M+1); ¹H-NMR (DMSO-d⁶, 400MHz): δ 1.66-1.90 (2H, m), 2.28 - 2.38 (1H, m), 2.44 - 2.54 (2H, m), 2.63 - 2.82 (2H, m), 3.05 - 3.25 (3H, m), 3.85 (2H, s), 6.29 (1H, d, *J*=6.4 Hz), 7.06 - 7.16 (1H, m), 7.25 - 7.35 (2H, m), 7.39 (1H, dd, *J*=7.8, 6.4 Hz) ppm. NH proton is not apparent.

The following compounds are prepared in a similar manner to Example 1 from their respective intermediates which maybe prepared by the methods described. The table indicates whether the L-(+)-tartaric acid salt (tart) or hemitartrate salt (hemitart.) is made.

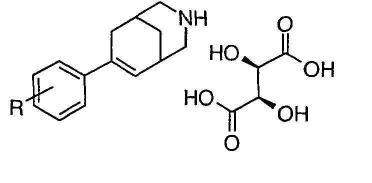

| Example / salt form | R | Data |
|---|---|---|
| **2** (tart) | 3,4-diF | Mass spectrum (m/z): 236 (M+1); ¹H-NMR (DMSO-d⁶, 400MHz): δ 1.61 - 1.93 (2H, m), 2.29 - 2.50 (3H, m), 2.63 - 2.81 (2H, m), 3.07 - 3.23 (3H, m), 3.88 (2H, s), 6.24 (1H, d, *J*=6.4 Hz), 7.27 - 7.35 (1H, m), 7.36 - 7.47 (1H, m), 7.49 - 7.58 (1H, m) ppm. NH proton is not apparent. |
| **3** | 3-F, 5-Br | Mass spectrum (m/z): 296 and 298 (M+2); 1H NMR (CDCl₃, 400 MHz) δ ppm 1.30 - 1.54 (1 H, m), 1.77 - 1.88 (2 H, m), 1.99 - 2.05 (1 H, m, *J*=2.7 Hz), 2.30 (1 H, d, *J*=17.9 Hz), 2.43 - 2.48 (1 H, m, *J*=3.9 Hz), 2.68 - 2.75 (2 H, m), 2.89 (1 H, dd, *J*=13.1, 2.6 Hz), 2.95 (1 H, d, *J*=2.2 Hz), 6.25 (1H, d, *J*=6.6 Hz), 7.04 - 7.17 (2 H, m), 7.39 (1 H, s). NH proton is not apparent. |
| **4** (tart) | 3,5-diF | Mass spectrum (m/z): 236 (M+1); ¹H-NMR (DMSO-d⁶, 400MHz): δ 1.63 - 1.73 (1H, m), 1.82 - 1.91 (1H, m), 2.41 - 2.53 (3H, m), 2.64 - 2.82 (2H, m), 3.02 - 3.24 (3H, m), 3.92 (2H, s), 6.35 (1H, d, *J*=6.4 Hz), 7.09 - 7.25 (3H, m) ppm. NH proton is not apparent. |
| **5** (tart) | 3,4,5-triF | Mass spectrum (m/z): 254 (M+1); ¹H-NMR (DMSO-d⁶, 400MHz): δ 1.77 (2H, dd, *J*=76.4, 12.1 Hz), 2.29 - 2.49 (3H, m), 2.65 - 2.80 (2H, m), 3.07 - 3.23 (3H, m), 3.90 (2H, s), 6.30 (1H, d, *J*=6.1 Hz), 7.34 - 7.47 (2H, m) ppm. NH proton is not apparent. |
| **6** (tart) | 2-F | Mass spectrum (m/z): 218 (M+1); ¹H-NMR (DMSO-d⁶, 400MHz): δ 1.67 - 1.92 (2H, m), 2.27 - 2.51 (3H, m), 2.63 - 2.85 (2H, m), 3.10 - 3.27 (3H, m), 3.92 (2H, s), 6.06 (1H, d, *J*=6.4 Hz), 7.10 - 7.24 (2H, m), 7.25 - 7.36 (1H, m), 7.46 - 7.58 (1H, m) ppm. NH proton is not apparent. |
| **7** (tart) | 3-CN | Mass spectrum (m/z): 225 (M+1); ¹H-NMR (DMSO-d⁶_{.} 400MHz): δ 1.71 ( H, d, *J*=12.5 Hz), 1.88 (1H, d, *J*=12.5 Hz), 2.28 - 2.54 (3H, m), 2.66 - 2.85 (2H, m), 3.08 - 3.25 (3H, m), 3.90 (2H, s), 6.33 (1H, d, *J*=6.1 Hz), 7.57 (1H, t, *J*=7.8 Hz), 7.75 (1H, d, *J*=7.6 Hz), 7.81 (1H, d, *J*=8.1 Hz), 7.92 (1H, s) ppm. NH proton is not apparent. |
| **8** | 3-F, 5-CF₃ | Mass spectrum (m/z): 286 (M+1); 1H NMR (CDCl₃, 400 MHz) δ ppm 1.42 - 1.54 (1 H, m), 1.79 - 1.90 (2 H, m), 2.06 (1 H, s), 2.31 - 2.41 (1 H, m), 2.49 (1 H, s), 2.71 - 2.80 (2 H, m), 2.91 (1 H, dd, *J*=13.1, 2.3 Hz), 2.97 (1 H, s), 6.33 (1 H, d, *J*=6.6 Hz), 7.20 (1 H, d, *J*=8.3 Hz), 7.33 (1 H, d, *J*=10.0 Hz), 7.51 (1 H, s). NH proton is not apparent. |
| **9** | 3-F, 5-OMe | 1H NMR (CDCl₃, 400 MHz) δ ppm 1.38 - 1.49 (1 H, m), 1.83 (2 H, ddd, *J*=5.4, 2.6, 2.3 Hz), 1.98 - 2.04 (1 H, m), 2.32 (1 H, d, *J*=17.9 Hz), 2.42 - 2.46 (1 H, m), 2.68 - 2.73 (1 H, m), 2.73 - 2.79 (1 H, m), 2.88 (1 H, dd, *J*=13.2, 2.4 Hz), 2.94 (1 H, d, *J*=2.4 Hz), 3.82 (3 H, s), 6.22 (1 H, d, *J*=6.4 Hz), 6.52 (1 H, dt, *J*=10.5, 2.2 Hz), 6.73 - 6.77 (1 H, m), 6.78 (1 H, t, *J*=2.1 Hz). NH proton is not apparent. |
| **10** | 3-F, 5-ethoxy | Mass spectrum (m/z): 262 (M+1); 1H NMR (400 MHz, DMSO-*d₆*) δ ppm 1.32 (t, *J*=6.97 Hz, 3 H) 1.68 (d, *J*=11.98 Hz, 1 H) 1.85 (d, *J*=12.47 Hz, 1 H) 2.32 (s, 1 H) 2.45 (d, *J*= 18.34 Hz, 1 H) 2.67 (s, 1 H) 2.74 (dd, *J*= 18.46, 6.72 Hz, 1 H) 3.02 - 3.11 (m, 3 H) 3.14 - 3.22 (m, 1 H) 4.06 (q, *J*=6.85 Hz, 2 H) 6.25 (d, *J*=6.36 Hz, 1 H) 6.73 (dt, *J*=11.00, 2.20 Hz, 1 H) 6.83 - 6.89 (m, 2 H). NH proton not apparent. |
| **11** | 3-F, 5-(3-methylisoxazol -5-yl) | Mass spectrum (m/z): 299 (M+1); 1H NMR (400 MHz, *DMSO-d₆*) δ ppm 1.72 (1 H, d, *J*=12.2 Hz), 1.88 (1 H, d, *J*=12.5 Hz), 2.30 (3 H, s), 2.35 (1 H, s), 2.53 - 2.57 (1 H, m), 2.72 (1 H, s), 2.83 (1 H, dd, *J*=18.2, 6.7 Hz), 3.05 - 3.13 (3 H, m), 3.17 - 3.24 (1 H, m), 6.39 (1 H, d, *J*=6.4 Hz), 7.03 (1 H, s), 7.43 (1 H, d, *J*=10.5 Hz), 7.62 (1 H, d, *J*=8.6 Hz), 7.77 (1 H, s). NH proton not apparent. |
| **12** (tart.) | 3-F, 5-(NMe-1,2,4-triazole) | Mass spectrum (m/z) 299 (M+1); 1H NMR (*DMSO-d₆,* 400 MHz) δ ppm 1.73 (1 H, s), 1.84 - 1.91 (1 H, m), 2.33 (1 H, d, *J*=1.7 Hz), 2.50 - 2.55 (2 H, m), 2.83 (1 H, dd, *J=18.7,* 6.7 Hz), 3.03 - 3.11 (1 H, m), 3.16 - 3.22 (1 H, m), 3.82 (2 H, s), 4.00 (3 H, s), 6.39 (1 H, d, *J*=6.4 Hz), 7.47 - 7.57 (2 H, m), 7.69 (1 H, t, *J*=1.5 Hz), 8.03 (1 H, s). NH proton is not apparent. |
| **13** (tart.) | 3-F, 5-(NMe-2-pyrrole) | Mass spectrum (m/z): 297 (M+1); 1H NMR (*DMSO-d₆,* 400 MHz) δ ppm 0.78 - 1.13 (1 H, m), 1.20 - 1.63 (1 H, m), 1.73 (1 H, s), 1.88 (1 H, d, *J*=12.5 Hz), 2.27 - 2.41 (1 H, m), 2.54 (1 H, s), 2.64 - 2.74 (1 H, m), 2.82 (1 H, dd, *J*= 18.3, 6.4 Hz), 3.04 - 3.12 (1 H, m), 3 .17 - 3.23 (1 H, m), 3.68 (3 H, s), 3.84 - 3.90 (2 H, m), 6.04 - 6.09 (1 H, m), 6.26 (1 H, dd, *J*=3.7, 2.0 Hz), 6.33 (1 H, d, *J*=6.4 Hz), 6.83 - 6.89 (1 H, m), 7.14 - 7.24 (2 H, m), 7.35 (1 H, s). NH proton is not apparent. |
| **14** (tart.) | 3-F, 5-(2-thiazole) | Mass spectrum (m/z): 301 (M+1); 1H NMR (*DMSO-d₆,* 400 MHz) δ ppm 1.73 (1 H, d, *J*=12.2 Hz), 1.88 (1 H, d, *J*=12.2 Hz), 2.36 (1 H, s), 2.49 - 2.53 (2 H, m), 2.72 (1 H, s), 2.83 (1 H, dd, *J*=18.3, 6.6 Hz), 3.05 - 3.14 (2 H, m), 3.19 - 3.27 (1 H, m), 3.87 (2 H, s), 6.37 (1 H, d, *J*=6.4 Hz), 7.42 (1 H, ddd, *J*=10.1, 2.2, 1.8 Hz), 7.59 - 7.70 (1 H, m), 7.83 - 7.89 (2 H, m), 7.97 (1 H, d, *J*=3.2 Hz). NH proton is not apparent. |
| **15** (tart.) | 3-F,5-(5-[1,2,4-oxadiazole]) | Mass spectrum (m/z): 286 (M+1); 1H NMR (*DMSO-d₆,* 400 MHz) δ ppm 1.71 (1 H, d, *J*=12.5 Hz), 1.88 (1 H, d, *J*=12.2 Hz), 2.38 (1 H, d, *J*=1.2 Hz), 2.47 - 2.53 (1 H, m), 2.54 (1 H, s), 2.71 - 2.83 (2 H, m), 3.06 - 3.17 (2 H, m), 3.26 (1 H, d, *J*=12.7 Hz), 4.23 (2 H, s), 6.29 (1 H, d, *J*=6.4 Hz), 7.30 (1 H, ddd, *J*=10.3, 2.2, 2.0 Hz), 7.51 (1 H, dd, *J*=9.7, 1.3 Hz), 7.93 (1 H, s). NH and oxadiazole CH are not apparent. |
| **16** (tart.) | 3-F, 5-acetyl | Mass spectrum (m/z): 260 (M+1); 1H NMR (*DMSO-d₆,* 400 MHz) δ ppm 1.73 (1 H, s), 1.88 (1 H, d, *J*=12.2 Hz), 2.35 (1 H, s), 2.49 - 2.52 (3 H, m), 2.71 (1 H, s), 2.81 (1 H, dd, *J*=18.2, 6.2 Hz), 3.08 (2 H, d, *J*=2.2 Hz), 3.11 (1 H, d, *J*=3.9 Hz), 3.21 (1 H, d, *J*=13.9 Hz), 3.85 (2 H, s), 6.37 (1 H, d, *J*=6.4 Hz), 7.51 - 7.61 (1 H, m), 7.62 - 7.72 (1 H, m), 7.86 (1 H, d, *J*=1.5 Hz). NH proton is not apparent. |
| **17** | 3-F, 5-(1H-imidazol-1-yl) | 1H NMR (*MeOD,* 400 MHz) δ ppm 1.84 - 1.96 (2 H, m), 2.09 - 2.15 (1 H, m), 2.51 - 2.56 (2 H, m), 2.80 - 2.84 (1 H, m), 2.85 - 2.90 (1 H, m), 2.93 (2 H, dd, *J*=14.5, 2.3 Hz), 2.99 - 3.06 (1 H, m), 6.41 (1 H, d, *J*=6.4 Hz), 7.17 (1 H, s), 7.26 - 7.34 (2 H, m), 7.49 - 7.57 (1 H, m), 7.66 (1 H, t, *J*=1.5 Hz), 8.24 (1 H, s). NH is proton not apparent. |
| **18** | 3-F, 5-(2,6-pyrimidine) | Mass spectrum (m/z): 296(M+1); 1H NMR (*MeOD,* 400 MHz) δ ppm 1.88 - 2.00 (3 H, m), 2.27 (1 H, d, *J*=3.7 Hz), 2.62 - 2.68 (1 H, m), 2.87 - 2.98 (2 H, m), 3.00 - 3.09 (2 H, m), 3.12 - 3.18 (1 H, m), 6.43 (1 H, d, *J*=6.4 Hz), 7.39 - 7.45 (2 H, m), 7.99 - 8.05 (1 H, m), 8.43 (1 H, s), 8.88 (2 H, t, *J*=4.8 Hz). NH proton is not apparent. |
| **19** | 3-F, 5-(4-pyridyl) | Mass spectrum (m/z): 295 (M+1); 1H NMR (CDCl₃, 400 MHz) δ ppm 1.44 - 1.56 (2 H, m), 1.81 - 1.91 (2 H, m), 2.06 (1 H, s), 2.40 (1 H, d, *J*=17.9 Hz), 2.49 (1 H, s), 2.71 - 2.80 (1 H, m), 2.92 (1 H, dd, *J*=13.1, 2.3 Hz), 2.95 - 2.99 (1 H, m), 6.33 (1 H, d, *J*=6.4 Hz), 7.20 - 7.26 (2 H, m), 7.46 - 7.52 (3 H, m), 7.64 - 7.74 (1 H, m), 8.67 - 8.70 (1 H, m). NH proton is not apparent. |
| **20** (tart.) | 3-F, 5-(3-furan) | Mass spectrum (m/z): 284 (M+1); 1H NMR (*DMSO-d₆,* 400 MHz) δ ppm 1.72 (1 H, d, *J*=12.0 Hz), 1.88 (1 H, d, *J*=12.2 Hz), 2.34 (1 H, s), 2.47 - 2.53 (1 H, m), 2.70 (1 H, s), 2.81 (1 H, dd, *J*=18.2, 6.5 Hz), 3.07 (2 H, d, *J*=2.0 Hz), 3.11 (1 H, d, *J*=2.9 Hz), 3.15 - 3.23 (1 H, m), 3.84 (2 H, s), 6.36 (1 H, d, *J*=6.4 Hz), 7.05 (1 H, d, *J*=1.0 Hz), 7.18 (1 H, dt, *J*=10.7,1.9 Hz), 7.41 (1 H, ddd, *J*=9.7, 2.2, 1.8 Hz), 7.55 (1 H, s), 7.76 (1 H, t, *J*=1.7 Hz), 8.30 (1 H, s). NH proton is not apparent. |
| **21** | 3-CN, 5-pyridin-4-yl | Mass spectrum (m/z): 302 (M+1); 1H NMR (400 MHz, DMSO-*d₆*) δ ppm 1.74 (d, *J*=11.98 Hz, 1 H) 1.90 (d, *J*=12.23 Hz, 1 H) 2.37 (s, 1 H) 2.57 (d, *J*=18.34 Hz, 1 H) 2.73 (s, 1 H) 2.90 (dd, *J*=18.22, 6.48 Hz, 1 H) 3.08 - 3.15 (m, 3 H) 3.18 - 3.25 (m, 1 H) 6.45 (d, *J*=6.36 Hz, 1 H) 7.83 - 7.86 (m, 2 H) 8.01 (s, 1 H) 8.16 (t, *J*=1.71 Hz, 1 H) 8.23 (s, 1 H) 8.69 - 8.74 (m, 2 H). NH proton not apparent. |
| **22** | 3-F, 5-CONH₂ | Mass spectrum (m/z): 261.2 |
| **23** | 3-F, 5-CONMe₂ | Mass spectrum (m/z): 289.2 |
| **24** | 3-F, 5-CO₂H | Mass spectrum (m/z): 262.2 |
| **25** | 3-F, 5-(1H-[1,3,4]triazol e) | Mass spectrum (m/z): 285.2 |
| **26** | 3-F, 5-isoxazole | Mass spectrum (m/z): 285.1 |
| **27** | 3-F, 5-(2H-[1,2,4]triazol e) | Mass spectrum (m/z): 285.1 |
| **28** | 3-F, 5-(5-methyl-[1,2,4]oxadia zole) | Mass spectrum (m/z): 300.1 |
| **29** | 3-F, 5-(3-methyl-[1,2,4]oxadia zole) | Mass spectrum (m/z): 316.1 |
| **30** | 3-CN, 5-(3H-imidazole) | Mass spectrum (m/z): 291.1 |
| **31** | 3-CN, 5-ethoxy | Mass spectrum (m/z): 269.1 |
| **32** | 3-Cyclopropyl methoxy, 5-F | Mass spectrum (m/z): 288.2 |
| **33** | 3-Difluorometh oxy, 5-F | Mass spectrum (m/z): 284.1 |
| **34** | 3-F, 5-pyridin-3-yl | Mass spectrum (m/z): 295.1 |
| **35** | 3F, 5-(2-fluoroethoxy) | Mass spectrum (m/z): 280.1 |
| **36** | 3-(2,2-Difluoroethoxy), 5-F | Mass spectrum (m/z): 298.1 |
| **37** | 3-F, 5(2,2,2-trifluoroethoxy) | Mass spectrum (m/z): 316.1 |
| **38** | 3-CN, 5-(2-fluoroethoxy) | Mass spectrum (m/z): 287.2 |
| **39** | 3-CN, 5-(2,2,2-trifluoroethoxy) | Mass spectrum (m/z): 323.1 |
| **40** | 3-F, 5-phenyl | Mass spectrum (m/z): 294.2 |
| **41** | 3-F, 5-phenoxy | Mass spectrum (m/z): 310.1 |

### Example 42:

### 7-(3,4-Difluoro-5-methoxy-phenyl)-3-methyl-3-aza-bicyclo[3.3.1]non-6-ene

*tert*-Butyl 7-(3,4-difluoro-5-methoxy-phenyl)-3-azabicyclo[3.3.1]non-6-ene-3-carboxylate (Intermediate 22, 0.04g, 0.15mol) is dissolved in dry Et₂O and LiAlH₄ (0.3mL as a 1 M solution in THF) is added at ambient temperature. The reaction mixture is stirred at ambient temperature overnight. Tlc analysis (1:1 hex:EtOAc) indicated starting material is still present. Additional LiALH₄ is added (0.9mL as a 1 M solution in THF) and the reaction is stirred at ambient temperature overnight. The reaction mixture is quenched by the sequential addition of water, 15% aqueous NaOH and water. The resulting suspension is absorbed onto a SCX-2 cartridge^{®} (5g). The ion exchange cartridge is washed with MeOH, and the product then eluted with MeOH / NH₃ (7N). The eluate is concentrated *in vacuo* to give a colourless oil. This is further purified by chromatography to obtain pure product (26mg, 0.093 mmol). Mass spectrum (m/z): M+1=280.2 ; 1H NMR (CDCl₃, 400 MHz) δ ppm 1.48 (1 H, s), 1.78 - 1.88 (2 H, m), 2.02 (1 H, s), 2.30 (1 H, d, *J*=18.1 Hz), 2.45 (1 H, d, *J*=2.7 Hz), 2.67 - 2.75 (2 H, m), 2.89 (1 H, dd, *J*=13.2, 2.4 Hz), 2.95 (1 H, d), 3.91 - 3.95 (3 H, m), 6.16 (1 H, d, *J*=6.6 Hz), 6.81 - 6.89 (1 H, m), 7.26 (1 H, s). NH proton is not apparent.

### Example 43 tert-Butyl 7-(3,5-difluorophenyl)-3-azabicyclo[3.3.1]nonane

Mass spectrum (m/z): 238 (M+1); ¹H-NMR (DMSO-d⁶, 400MHz): δ 1.37 (1H, d, *J*=13.2 Hz), 1.48 - 1.61 (2H, m), 1.89 - 1.99 (1H, m), 2.00 - 2.14 (2H, m), 2.18 - 2.28 (2H, m), 2.74 - 3.11 (5H, m), 3.98 (2H, s), 6.96 - 7.05 (1H, m), 7.15 - 7.24 (2H, m) ppm. NH proton not apparent.

### The chromatographic resolution of compounds

Compounds prepared by these methods are racemic. These may be separated into their component isomers using the following method: The racemic free base is dissolved in a minimum amount of IPA. The filtered solution is injected onto an AD-H column (a 21.2 x 250mm column where the stationary phase is coated with an appropriate polysaccharide), and the compound is resolved by elution with super critical fluid (65% CO₂ (100bar), 35% IPA containing 0.2% dimethylethylamine), at 35°C, at a rate of 70mL/min.

## Claims

1. A compound according to formula I or a pharmaceutically acceptable salt thereof: 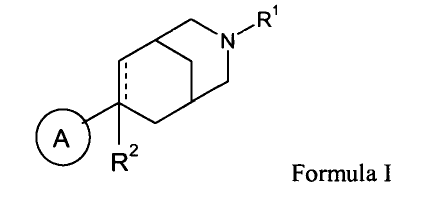 wherein,
R¹ is hydrogen, C₁₋₆alkoxycarbonyl, C₁₋₆alkyl, benzyloxycarbonyl, cyanoC₁₋₆alkyl, dihydro-3-pyridinylcarbonyl, hydroxy, hydroxyC₁₋₆alkyl, phenoxycarbonyl, -NR³R⁴, (NR³R⁴)C₁₋₄alkyl and (NR³R⁴)carbonylC₁₋₄alkyl;
R² is present as H or OH;
⁻⁻⁻⁻⁻ is an optional double bond, wherein when ⁻⁻⁻⁻⁻ is a double bond R² is absent;
A is an aryl selected from phenyl, naphthyl, indenyl, indanyl, azulenyl and tetrahydronapthyl,
wherein said aryl is optionally substituted with one, two, three or four substituents independently selected from C₂₋₆alkenyl, C₁₋₆ alkoxy, C₁₋₆alkoxyC₁₋₄alkoxy, C₁₋₆alkoxyC₁₋₄alkyl, C₁₋₆alkoxycarbonyl, C₁₋₆alkoxycarbonylC₁₋₄alkyl, C₁₋₆alkyl, C₁₋₆alkylcarbonyl, C₁₋₆alkylcarbonyloxy, C₁₋₆alkylthio, C₂₋₆alkynyl, carboxy, carboxyC₁₋₆alkyl, cyano, cyanoC₁₋₆alkyl, C₃₋₆cycloalkyl, C₃₋₆cycloalkylC₁₋₄alkyl, haloC₃₋₆cycloalkylC₁₋₄alkyl, C₃₋₆cycloalkylC₁₋₄alkoxy, haloC₃₋₆cycloalkylC₁₋₄alkoxy, formyl, formylC₁₋₆alkyl, haloC₁₋₆alkoxy, haloC₁₋₆alkyl, halogen, hydroxy, hydroxyC₁₋₆alkyl, mercapto, mercaptoC₁₋₆alkyl, nitro, triphenylmethyl (trityl), a heteroaryl optionally substituted by one, two or three methyl substituents, wherein said heteroaryl is selected from furyl, thienyl, pyrrolyl, pyrazolyl, imidazolyl, triazolyl, oxazolyl, isoxazolyl, thiazolyl, thiadiazolyl, isothiazolyl, oxadiazolyl, oxatriazolyl, pyridinyl, pyridazinyl, pyrimidinyl, pyrazinyl, triazinyl and tetrazolyl, -C(NH)NR³R⁴, -NR³R⁴, -C₁₋₄alkyl (NR³R⁴), -CO(NR³R⁴), -C₁₋₄alkylCO(NR³R⁴), -S(O)₂ NR³R⁴, - NR⁵S(O)₂ R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂R⁵, phenyl and phenyl(CH₂)ₙO, wherein said phenyl or phenyl(CH₂)ₙO is optionally substituted with one, two or three substituents independently selected from chlorine, fluorine, cyano, C₁₋₃alkyl and C₁₋₃alkoxy, wherein said C₁₋₃alkyl or C₁₋₃alkoxy is optionally substituted with 1 to 3 fluorines; or where two substituents on adjacent carbon atoms of said aryl together optionally represent - OCH₂CH₂-, -OCH₂CH(OH)-, -OCH₂CH₂O- or -OCH₂O-;
R³ and R⁴ are independently selected from hydrogen, C₁₋₄alkyl and C₁₋₄alkylcarbonyl; R⁵, R⁶ and R⁷ are independently selected from hydrogen, C₁₋₄alkyl, phenyl and phenylC₁₋₄alkyl;
n is 0, 1 or 2.

2. The compound according to claim 1, wherein A is a phenyl optionally substituted with one, two, three or four substituents independently selected from C₂₋₆alkenyl, C₁₋₆alkoxy, C₁₋₆alkoxyC₁₋₄alkoxy, C₁₋₆alkoxyC₁₋₄alkyl, C₁₋₆ alkoxycarbonyl, C₁₋₆alkoxycarbonylC₁₋₄alkyl, C₁₋₆alkyl, C₁₋₆alkylcarbonyl, C₁₋₆alkylcarbonyloxy, C₁₋₆alkylthio, C₂₋₆alkynyl, carboxy, carboxyC₁₋₆alkyl, cyano, cyanoC₁₋₆alkyl, C₃₋₆cycloalkyl, C₃₋₆cycloalkylC₁₋₄alkyl, formyl, formylC₁₋₆alkyl, haloC₁₋₆alkoxy, haloC₁₋₆alkyl, halogen, hydroxy, hydroxyC₁₋₆alkyl, mercapto, mercaptoC₁₋₆alkyl, nitro, triphenylmethyl (trityl), furyl, thienyl, pyrrolyl, pyrazolyl, imidazolyl, triazolyl, oxazolyl, isoxazolyl, thiazolyl, thiadiazolyl, isothiazolyl, oxadiazolyl, oxatriazolyl, pyridinyl, pyridazinyl, pyrimidinyl, pyrazinyl, triazinyl, tetrazolyl, -C(NH)NR³R⁴, -NR³R⁴, -C₁₋₄alkyl (NR³R⁴), -CO(NR³R⁴), -C₁₋₄alkylCO(NR³R⁴), -S(O)₂ NR³R⁴, -NR⁵S(O)₂ R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂ R⁵, phenyl and phenyl(CH₂)ₙO, wherein said phenyl or phenyl(CH₂)ₙO is optionally substituted with one, two or three substituents independently selected from chlorine, fluorine and cyano; or where two substituents on adjacent carbon atoms of said aryl together optionally represent -OCH₂CH₂-, -OCH₂CH(OH)-, -OCH₂CH₂O- or -OCH₂O-.

3. The compound according to claim 1 or claim 2, wherein
R¹ is H, C₁₋₃alkoxycarbonyl, C₁₋₃alkyl, cyanoC₁₋₃alkyl, hydroxy or hydroxyC₁₋₃alkyl;
A is a phenyl optionally substituted with one, two or three substituents independently selected from amide, C₂₋₄alkenyl, C₁₋₃alkoxy, C₁₋₃alkoxyC₁₋₃alkyl,
C₁₋₃alkoxycarbonyl, C₁₋₃alkyl, C₁₋₃alkylcarbonyl, C₁₋₃alkylcarbonyloxy, C₁₋₃alkylthio, C₂₋₄alkynyl, carboxy, carboxyC₁₋₃alkyl, cyano, cyanoC₁₋₃alkyl, formyl, formylC₁₋₃alkyl, haloC₁₋₃alkoxy, haloC₁₋₃alkyl, halogen, hydroxy, hydroxyC₁₋₃alkyl, mercapto, mercaptoC₁₋₃alkyl, nitro, furyl, thienyl, pyrrolyl, pyrazolyl, imidazolyl, triazolyl, oxazolyl, isoxazolyl, thiazolyl, thiadiazolyl, isothiazolyl, oxadiazolyl, oxatriazolyl, pyridinyl, pyridazinyl, pyrimidinyl, pyrazinyl, triazinyl, tetrazolyl, phenyl and phenyl(CH₂)ₙO, wherein said phenyl or phenyl(CH₂)ₙO is optionally substituted with one, two or three substituents independently selected from chlorine, fluorine and cyano, wherein n=0, 1 or 2.

4. The compound according to claim 3, wherein
R¹ is H, C₁₋₃alkyl or hydroxy.

5. The compound according to any one of claims 1 to 4, wherein
R¹ is H or methyl;
A is a phenyl optionally substituted with one, two or three substituents
independently selected from cyano, fluorine, chlorine, bromine, methyl, ethyl, isopropyl, methoxy, ethoxy, isopropyloxy, benzyloxy, trifluoromethyl, difluoromethoxy, trifluoromethoxy, phenoxy, 4-chlorophenoxy, 4-cyanophenoxy and 4-fluorophenoxy.

6. The compound according to claim 5, wherein
A is a phenyl group selected from 2-fluorophenyl, 3-fluorophenyl,
3,5-difluorophenyl, 3,4-difluorophenyl, 3,4,5-trifluorophenyl, 3-cyanophenyl and 3-methoxyphenyl.

7. The compound according to claim 1 wherein said compound has the Formula III: 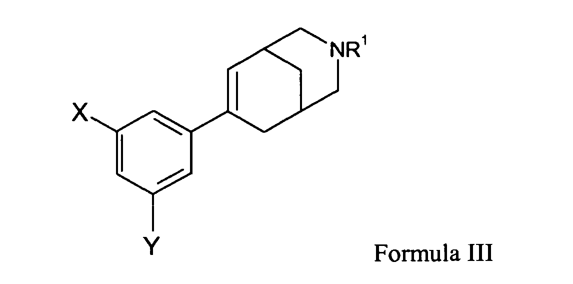 wherein X is selected from
a C₁₋₆alkyl, C₂₋₆alkenyl, C₁₋₆alkoxy, C₁₋₆alkoxyC₁₋₄alkoxy, C₁₋₆alkoxyC₁₋₄alkyl, C₁₋₆alkoxycarbonyl, C₁₋₆alkoxycarbonylC₁₋₄alkyl, C₁₋₆alkylcarbonyl, C₁₋₆alkylcarbonyloxy, C₁₋₆alkylthio, C₂₋₆alkynyl, carboxy, carboxyC₁₋₆alkyl, cyano, cyanoC₁₋₆alkyl, C₃₋₆cycloalkyl, C₃₋₆cycloalkylC₁₋₄alkyl, C₃₋₆cycloalkylC₁₋₄alkoxy, wherein said C₃₋₆cycloalkylC₁₋₄alkyl or C₃₋₆cycloalkylC₁₋₄alkoxy is optionally substituted with 1 to 3 halogen atoms; formylC₁₋₆alkyl, haloC₁₋₆alkoxy, haloC₁₋₆alkyl, halogen, hydrogen, hydroxy, hydroxyC₁₋₆alkyl, carboxy, mercapto, mercaptoC₁₋₆alkyl, nitro, triphenylmethyl (trityl), -C(NH)NR³R⁴, -NR³R⁴, -C₁₋₄alkyl (NR³R⁴), -CO(NR³R⁴), -C₁₋₄alkylCO(NR³R⁴), -S(O)₂ NR³R⁴, -NR⁵S(O)₂ R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂R⁵, a heteroaryl optionally substituted by one, two or three methyl substituents, wherein said heteroaryl is selected from furyl,
thienyl, pyrrolyl, pyrazolyl, imidazolyl, triazolyl, oxazolyl, isoxazolyl, thiazolyl, thiadiazolyl, isothiazolyl, oxadiazolyl, oxatriazolyl, pyridinyl, pyridazinyl, pyrimidinyl, pyrazinyl, triazinyl and tetrazolyl, phenyl and phenyl(CH₂)ₙO, wherein said phenyl or phenyl(CH₂)ₙO is optionally substituted with one, two or three substituents independently selected from chlorine, fluorine, cyano, C₁₋₃alkyl and C₁₋₃alkoxy wherein said C₁₋₃alkyl or C₁₋₃alkoxy is optionally substituted with 1 to 3 fluorines;
Y is selected from cyano, hydrogen, fluoro, chloro and bromo;
R¹ is selected from hydrogen and C₁₋₄alkyl;
R³ and R⁴ are independently selected from hydrogen, C₁₋₄alkyl and C₁₋₄alkylcarbonyl;
R⁵, R⁶ and R⁷ are independently selected from hydrogen, C₁₋₄alkyl, phenyl and phenylC₁₋₄alkyl;

8. The compound according to claim 7, wherein
X is selected from C₁₋₃alkyl, C₂₋₄alkenyl, C₁₋₃alkoxy, C₁₋₃alkoxyC₁₋₃alkoxy, C₁₋₃alkoxyC₁₋₃alkyl, C₁₋₃alkoxycarbonyl, C₁₋₃alkoxycarbonylC₁₋₃alkyl, C₁₋₃alkylcarbonyl, C₁₋₃alkylcarbonyloxy, C₁₋₃alkylthio, C₂₋₄alkynyl, carboxy, cyano, cyanoC₁₋₃alkyl, cyclopropyl, cyclopropylC₁₋₃alkyl, cyclopropylC₁₋₃alkoxy, wherein said C₁₋₃alkyl, C₁₋₃alkoxy, cyclopropylC₁₋₃alkyl, cyclopropylC₁₋₃alkoxy is optionally substituted with 1 to 3 halogen atoms independently selected from fluorine and chlorine; formylC₁₋₃alkyl, halogen, hydrogen, hydroxy, hydroxyC₁₋₃alkyl, mercapto, mercaptoC₁₋₃alkyl, nitro, -C(NH)NR³R⁴, -NR³R⁴, -C₁₋₃alkyl (NR³R⁴), -CO(NR³R⁴), -C₁₋₃alkylCO(NR³R⁴), -S(O)₂ NR³R⁴, -NR⁵S(O)₂ R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵ -S(O)₂ R⁵, a heteroaryl optionally substituted by one, two or three methyl substituents, wherein said heteroaryl is selected from furyl, thienyl, pyrrolyl,
pyrazolyl, imidazolyl, triazolyl, oxazolyl, isoxazolyl, thiazolyl, thiadiazolyl, isothiazolyl, oxadiazolyl, pyridinyl, pyridazinyl, pyrimidinyl and pyrazinyl, phenyl and phenyl(CH₂)ₙO, wherein said phenyl or phenyl(CH₂)ₙO is optionally substituted with one, two or three substituents independently selected from fluorine, cyano, methyl, ethyl, methoxy and ethoxy, wherein said methyl, ethyl, methoxy or ethoxy is optionally substituted with 1 to 3 fluorines;
Y is selected from cyano, hydrogen, fluoro and chloro;
R¹ is selected from methyl and hydrogen;
R³ and R⁴ are independently selected from hydrogen, C₁₋₃alkyl and C₁₋₃alkylcarbonyl;
R⁵, R⁶ and R⁷ are independently selected from hydrogen and C₁₋₃alkyl;
n is 0 or 1.

9. The compound according to either claim 7 or claim 8, wherein
X is selected from hydrogen, fluoro, chloro, bromo, -CONH₂, acetyl, C₁₋₃alkyl, C₁₋₃alkoxy, cyclopropylC₁₋₃alkyl and cyclopropylC₁₋₃alkoxy, wherein said C₁₋₃alkyl, C₁₋₃alkoxy, cyclopropylC₁₋₃alkyl or cyclopropylC₁₋₃alkoxy is optionally substituted with 1 to 3 fluorines, a heteroaryl optionally substituted with a methyl, wherein said heteroaryl is selected from furyl, pyrrolyl, imidazolyl, triazolyl, isoxazolyl, oxadiazolyl, thiadiazolyl and pyridinyl;
Y is selected from cyano, hydrogen and fluoro, wherein X and Y do not equal hydrogen;
R¹ is hydrogen.

10. The compound according to any one of claims 7 to 9, wherein
X is selected from fluoro, methoxy, ethoxy, acetyl, -CONH₂, 2-fluoro-ethoxy, 2,2-difluoro-ethoxy, 2,2,2-trifluoro-ethoxy and cyclopropyl-methoxy or a heteroaryl selected from 1-imidazolyl, 5-isoxazolyl, 3-pyridinyl, 4-pyridinyl and 5-thiadiazolyl, wherein said heteroaryl is optionally substituted with a methyl;
Y is fluoro.

11. A pharmaceutical composition comprising a compound according to any one of claims 1 to 10.

12. A compound according to any one of claims 1 to 10 for use in therapy.

13. A compound according to any one of claims 1 to 10 for use in the treatment or prevention of one or more conditions in a human selected from inflammatory bowel disease, ulcerative colitis, pyoderma gangrenosum, Crohn's disease, irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotrophic lateral sclerosis (ALS), cognitive-dysfunction, hypertension, bulimia, anorexia, obesity, cardiac arrhythmias, gastric acid hypersecretion, ulcers, pheochromocytoma, progressive supranuclear palsy, chemical dependencies and addictions, dependencies on, or addictions to nicotine (or tobacco products), alcohol, benzodiazopines, barbiturates, opioids or cocaine, headache, migraine, stroke, traumatic brain injury (TBI), obsessive-compulsive disorder (OCD), psychosis, Huntington's chorea, tardive dyskinesia, hyperkinesia, dyslexia, schizophrenia, multi-infarct dementia, age-related cognitive decline, epilepsy, petit mal absence epilepsy, senile dementia of the Alzheimer's type (AD), Parkinson's disease (PD), attention deficit hyperactivity disorder (ADHD), attention deficit disorder (ADD), restless legs syndrome (RLS), mild cognitive impairment, cognitive enhancement in schizophrenia, drug induced extrapyramidal symptoms, conduct disorder, oppositional defiant disorder, anxiety in anxious smokers, cardiovascular risk in pregnancy, delayed ejaculation, emesis, diarrhoea, nicotine gum addiction, sleep prevention, ischemia and Tourette's Syndrome.

14. A compound according to any one of claims 1 to 10 for use in the treatment or prevention of pain in a human.

## Patentansprüche

1. Verbindung gemäß der Formel I oder ein pharmazeutisch akzeptables Salz hiervon: 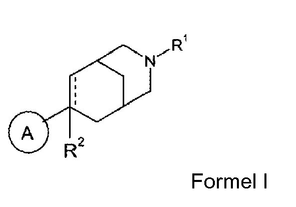 worin
R¹ für Wasserstoff, C₁₋₆-Alkoxycarbonyl, C₁₋₆-Alkyl, Benzyloxycarbonyl, Cyano-C₁₋₆-alkyl, Dihydro-3-pyridinylcarbonyl, Hydroxy, Hydroxy-C₁₋₆-alkyl, Phenoxycarbonyl, -NR³R⁴, (NR³R⁴)C₁₋₄-Alkyl und (NR³R⁴)-Carbonyl-C₁₋₄-alkyl steht;
R² für Wasserstoff oder -OH steht;
----- für eine optionale Doppelbindung steht, wobei, wenn ----- für eine Doppelbindung steht, R² fehlt;
A für ein Aryl steht, das aus Phenyl, Naphthyl, Indenyl, Indanyl, Azulenyl und Tetrahydronaphthyl ausgewählt ist,
wobei das Aryl optional mit einem, zwei, drei oder vier Substituenten substituiert ist, die unabhängig aus C₂₋₆-Alkenyl, C₁₋₆-Alkoxy, C₁₋₆-Alkoxy-C₁₋₄-alkoxy, C₁₋₆-Alkoxy-C₁₋₄-alkyl, C₁₋₆-Alkoxycarbonyl, C₁₋₆-Alkoxycarbonyl-C₁₋₄-alkyl, C₁₋₆-Alkyl, C₁₋₆-Alkylcarbonyl, C₁₋₆-Alkylcarbonyloxy, C₁₋₆-Alkylthio, C₂₋₆-Alkinyl, Carboxy, Carboxy-C₁₋₆-alkyl, Cyano, Cyano-C₁₋₆-alkyl, C₃₋₆-Cycloalkyl, C₃₋₆-Cycloalkyl-C₁₋₄-alkyl, Halogen-C₃₋₆-cycloalkyl-C₁₋₄-alkyl, C₃₋₆-Cycloalkyl-C₁₋₄-alkoxy, Halogen-C₃₋₆-cycloalkyl-C₁₋₄-alkoxy, Formyl, Formyl-C₁₋₆-alkyl, Halogen-C₁₋₆-alkoxy, Halogen-C₁₋₆-alkyl, Halogen, Hydroxy, Hydroxy-C₁₋₆-alkyl, Mercapto, Mercapto-C₁₋₆-alkyl, Nitro, Triphenylmethyl (Trityl), einem optional durch einem, zwei oder drei Methylsubstituenten substituierten Heteroaryl, wobei das Heteroaryl aus Furyl, Thienyl, Pyrrolyl, Pyrazolyl, Imidazolyl, Triazolyl, Oxazolyl, Isoxazolyl, Thiazolyl, Thiadiazolyl, Isothiazolyl, Oxadiazolyl, Oxatriazolyl, Pyridinyl, Pyridazinyl, Pyrimidinyl, Pyrazinyl, Triazinyl und Tetrazolyl ausgewählt ist, - C(NH)NR³R⁴, -NR³R⁴, -C₁₋₄-alkyl(NR³R⁴), -CO(NR³R⁴), -C₁₋₄-AlkylCO(NR³R⁴), S(O)₂NR³R⁴, NR⁵S(O)₂R⁶,
-C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂R⁵, Phenyl und Phenyl(CH₂)ₙO, wobei das Phenyl oder Phenyl(CH₂)ₙO optional mit einem, zwei oder drei Substituenten substituiert ist, die unabhängig aus Chlor, Fluor, Cyano, C₁₋₃-Alkyl und C₁₋₃-Alkoxy ausgewählt sind, wobei das C₁₋₃-Alkyl oder C₁₋₃-Alkoxy optional mit 1 bis 3 Fluoratomen substituiert ist; oder wobei zwei Substituenten an benachbarten Kohlenstoffatomen des Aryls zusammen optional für -OCH₂CH₂-, -OCH₂CH(OH)-, -OCH₂CH₂O- oder -OCH₂O-stehen, ausgewählt sind;
R³ und R⁴ unabhängig aus Wasserstoff, C₁₋₄-Alkyl und C₁₋₄-Alkylcarbonyl ausgewählt sind;
R⁵, R⁶ und R⁷ unabhängig aus Wasserstoff, C₁₋₄-Alkyl, Phenyl und Phenyl-C₁₋₄-alkyl ausgewählt sind; und
n für 0, 1 oder 2 steht.

2. Verbindung nach Anspruch 1, worin A für ein Phenyl steht, das optional mit einem, zwei, drei oder vier Substituenten substituiert ist, die unabhängig aus C₂₋₆-Alkenyl, C₁₋₆-Alkoxy, C₁₋₆-Alkoxy-C₁₋₄-alkoxy, C₁₋₆-Alkoxy-C₁₋₄-alkyl, C₁₋₆-Alkoxycarbonyl, C₁₋₆-Alkoxycarbonyl-C₁₋₄-alkyl, C₁₋₆-Alkyl, C₁₋₆-Alkylcarbonyl, C₁₋₆-Alkylcarbonyloxy, C₁₋₆-Alkylthio, C₂₋₆-Alkinyl, Carboxy, Carboxy-C₁₋₆-alkyl, Cyano, Cyano-C₁₋₆-alkyl, C₃₋₆-Cycloalkyl, C₃₋₆-Cycloalkyl-C₁₋₄-alkyl, Formyl, Formyl-C₁₋₆-alkyl, Halogen-C₁₋₆-alkoxy, Halogen-C₁₋₆-alkyl, Halogen, Hydroxy, Hydroxy-C₁₋₆-alkyl, Mercapto, Mercapto-C₁₋₆-alkyl, Nitro, Triphenylmethyl (Trityl), Furyl, Thienyl, Pyrrolyl, Pyrazolyl, Imidazolyl, Triazolyl, Oxazolyl, Isoxazolyl, Thiazolyl, Thiadiazolyl, Isothiazolyl, Oxadiazolyl, Oxatriazolyl, Pyridinyl, Pyridazinyl, Pyrimidinyl, Pyrazinyl, Triazinyl, Tetrazolyl, -C(NH)NR³R⁴, -NR³R⁴, -C₁₋₄-Alkyl(NR³R⁴),
-CO(NR³R⁴), -C₁₋₄-AlkylCO(NR³R⁴), -S(O)₂NR³R⁴, -NR⁵S(O)₂R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂R⁵, Phenyl und Phenyl(CH₂)ₙO, wobei das Phenyl oder Phenyl(CH₂)ₙO optional mit einem, zwei oder drei Substituenten substituiert sind, die unabhängig aus Chlor, Fluor und Cyano ausgewählt sind oder wobei zwei Substituenten an benachbarten Kohlenstoffatomen des Aryl zusammen optional für - OCH₂CH₂-, -OCH₂CH(OH)-, -OCH₂CH₂O- oder -OCH₂O- stehen, ausgewählt sind.

3. Verbindung nach Anspruch 1 oder 2, worin
R¹ für H, C₁₋₃-Alkoxycarbonyl, C₁₋₃-Alkyl, Cyano-C₁₋₃-alkyl, Hydroxy oder Hydroxy-C₁₋₃-alkyl steht;
A für ein Phenyl steht, das optional mit einem, zwei oder drei Substituenten substituiert ist, die unabhängig voneinander aus Amid, C₂₋₄-Alkenyl, C₁₋₃-Alkoxy, C₁₋₃-Alkoxy-C₁₋₃-alkyl, C₁₋₃-Alkoxycarbonyl, C₁₋₃-Alkyl, C₁₋₃-Alkylcarbonyl, C₁₋₃-Alkylcarbonyloxy, C₁₋₃-Alkylthio, C₂₋₄-Alkinyl, Carboxy, Carboxy-C₁₋₃-alkyl, Cyano, Cyano-C₁₋₃-alkyl, Formyl, Formyl-C₁₋₃-alkyl, Halogen-C₁₋₃-alkoxy, Halogen-C₁₋₃-alkyl, Halogen, Hydroxy, Hydroxy-C₁₋₃-alkyl, Mercapto, Mercapto-C₁₋₃-alkyl, Nitro, Furyl, Thienyl, Pyrrolyl, Pyrazolyl, Imidazolyl, Triazolyl, Oxazolyl, Isoxazolyl, Thiazolyl, Thiadiazolyl, Isothiazolyl, Oxadiazolyl, Oxatriazolyl, Pyridinyl, Pyridazinyl, Pyrimidinyl, Pyrazinyl, Triazinyl, Tetrazolyl, Phenyl und Phenyl-(CH₂)ₙO, wobei das Phenyl oder Phenyl-(CH₂)ₙO optional mit einem, zwei oder drei Substituenten substituiert sind, die unabhängig aus Chlor, Fluor und Cyano ausgewählt sind, wobei n für 0, 1 oder 2 steht, ausgewählt sind.

4. Verbindung nach Anspruch 3, worin
R¹ für H, C₁₋₃-Alkyl oder Hydroxy steht.

5. Verbindung nach einem der Ansprüche 1 bis 4, worin
R¹ für H oder Methyl steht;
A für ein Phenyl steht, das optional mit einem, zwei oder drei Substituenten substituiert ist, die unabhängig aus Cyano, Fluor, Chlor, Brom, Methyl, Ethyl, Isopropyl, Methoxy, Ethoxy, Isopropyloxy, Benzyloxy, Trifluormethyl, Difluormethoxy, Trifluormethoxy, Phenoxy, 4-Chlorphenoxy, 4-Cyanophenoxy und 4-Fluorphenoxy ausgewählt sind.

6. Verbindung nach Anspruch 5, worin
A für eine Phenylgruppe steht, die aus 2-Fluorphenyl, 3-Fluorphenyl, 3,5-Difluorphenyl, 3,4-Difluorphenyl, 3,4,5-Trifluorphenyl, 3-Cyanophenyl und 3-Methoxyphenyl ausgewählt ist.

7. Verbindung nach Anspruch 1, worin die Verbindung die Formel III besitzt: 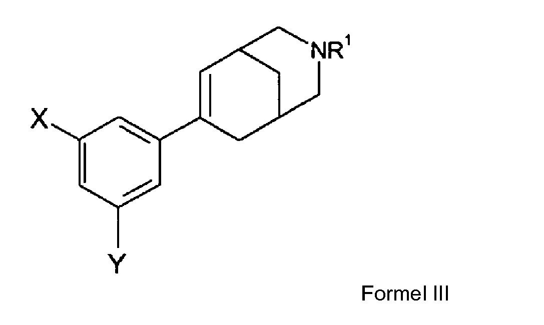 worin X aus
C₁₋₆-Alkyl, C₂₋₆-Alkenyl, C₁₋₆-Alkoxy, C₁₋₆-Alkoxy-C₁₋₄-alkoxy, C₁₋₆-Alkoxy-C₁₋₄-alkyl, C₁₋₆-Alkoxycarbonyl, C₁₋₆-Alkoxycarbonyl-C₁₋₄-alkyl, C₁₋₆-Alkylcarbonyl, C₁₋₆-Alkylcarbonyloxy, C₁₋₆-Alkylthio, C₂₋₆-Alkinyl, Carboxy, Carboxy-C₁₋₆-alkyl, Cyano, Cyano-C₁₋₆-alkyl, C₃₋₆-Cycloalkyl, C₃₋₆-Cycloalkyl-C₁₋₄-alkyl, C₃₋₆-Cycloalkyl-C₁₋₄-alkoxy, wobei das C₃₋₆-Cycloalkyl-C₁₋₄-alkyl oder C₃₋₆-Cycloalkyl-C₁₋₄-alkoxy optional mit 1 bis 3 Halogenatomen substituiert ist; Formyl-C₁₋₆-alkyl, Halogen-C₁₋₆-alkoxy, Halogen-C₁₋₆-alkyl, Halogen, Wasserstoff, Hydroxy, Hydroxy-C₁₋₆-alkyl, Carboxy, Mercapto, Mercapto-C₁₋₆-alkyl, Nitro, Triphenylmethyl (Trityl), -C(NH)NR³R⁴, -NR³R⁴, - C₁₋₄-Alkyl(NR³R⁴), -CO(NR³R⁴), -C₁₋₄-Alkyl-CO(NR³R⁴), S(O)₂NR³R⁴, -NR⁵S(O)₂R⁶, - C(NR⁵)NR⁶R⁷,
-CH₂C(NR⁵)NR⁶R⁷, -C(NOR⁵)R⁶, -C(NCN)R⁵, -C(NNR⁵R⁶)R⁷, -S(O)₂OR⁵, -S(O)₂⁵, einem optional mit einem, zwei oder drei Methylsubstituenten substituiertem Heteroaryl, wobei das Heteroaryl aus Furyl, Thienyl, Pyrrolyl, Pyrazolyl, Imidazolyl, Triazolyl, Oxazolyl, Isoxazolyl,
Thiazolyl, Thiadiazolyl, Isothiazolyl, Oxadiazolyl, Oxatriazolyl, Pyridinyl, Pyridazinyl, Pyrimidinyl, Pyrazinyl, Triazinyl und Tetrazolyl ausgewählt ist, Phenyl und Phenyl-(CH₂)ₙO, wobei das Phenyl oder Phenyl-(CH₂)ₙO optional mit einem, zwei oder drei Substituenten substituiert ist, die unabhängig voneinander aus Chlor, Fluor, Cyano, C₁₋₃-Alkyl und C₁₋₃-Alkoxy ausgewählt sind, wobei das C₁₋₃-Alkyl oder C₁₋₃-Alkoxy optional mit 1 bis 3 Fluoratomen substituiert ist, ausgewählt ist;
Y aus Cyano, Wasserstoff, Fluor, Chlor und Brom ausgewählt ist;
R¹ aus Wasserstoff und C₁₋₄-Alkyl ausgewählt ist;
R³ und R⁴ unabhängig aus Wasserstoff, C₁₋₄-Alkyl und C₁₋₄-Alkylcarbonyl ausgewählt sind; R⁵, R⁶ und R⁷ unabhängig aus Wasserstoff, C₁₋₄-Alkyl, Phenyl und Phenyl-C₁₋₄-alkyl ausgewählt sind.

8. Verbindung nach Anspruch 7, worin
X aus C₁₋₃-Alkyl, C₂₋₄-Alkenyl, C₁₋₃-Alkoxy, C₁₋₃-Alkoxy-C₁₋₃-alkoxy, C₁₋₃-Alkoxy-C₁₋₃-alkyl, C₁₋₃-Alkoxycarbonyl, C₁₋₃-Alkoxycarbonyl-C₁₋₃-Alkyl, C₁₋₃-Alkylcarbonyl, C₁₋₃-Alkylcarbonyloxy, C₁₋₃-Alkylthio, C₂₋₄-Alkinyl, Carboxy, Cyano, Cyano-C₁₋₃-alkyl, Cyclopropyl, Cyclopropyl-C₁₋₃-alkyl, Cyclopropyl-C₁₋₃-alkoxy, wobei das C₁₋₃-Alkyl, C₁₋₃-Alkoxy, Cyclopropyl-C₁₋₃-alkyl, Cyclopropyl-C₁₋₃-alkoxy optional mit 1 bis 3 Halogenatomen substituiert ist, die unabhängig aus Fluor und Chlor ausgewählt sind; Formyl-C₁₋₃-alkyl, Halogen, Wasserstoff, Hydroxy, Hydroxy-C₁₋₃-alkyl, Mercapto, Mercapto-C₁₋₃-alkyl, Nitro, -C(NH)NR³R⁴, -NR³R⁴, -C₁₋₃-Alkyl(NR³R⁴), -CO(NR³R⁴), -C₁₋₃-AlkylCO(NR³R⁴), S(O)₂NR³R⁴, -NR⁵S(O)₂R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂R⁵, einem optional mit einem, zwei oder drei Methylsubstituenten substituiertem Heteroaryl, wobei das Heteroaryl aus Furyl, Thienyl, Pyrrolyl,
Pyrazolyl, Imidazolyl, Triazolyl, Oxazolyl, Isoxazolyl, Thiazolyl, Thiadiazolyl, Isothiazolyl, Oxadiazolyl, Pyridinyl, Pyridazinyl, Pyrimidinyl und Pyrazinyl ausgewählt ist, Phenyl und Phenyl-(CH₂)ₙO, wobei das Phenyl oder Phenyl-(CH₂)ₙO optional mit einem, zwei oder drei Substituenten substituiert ist, die unabhängig voneinander aus Fluor, Cyano, Methyl, Ethyl, Methoxy und Ethoxy ausgewählt sind, wobei das Methyl, Ethyl, Methoxy oder Ethoxy optional mit 1 bis 3 Fluoratomen substituiert ist, ausgewählt ist;
Y aus Cyano, Wasserstoff, Fluor und Chlor ausgewählt ist;
R¹ aus Methyl und Wasserstoff ausgewählt ist;
R³ und R⁴ unabhängig aus Wasserstoff, C₁₋₃-Alkyl und C₁₋₃-Alkylcarbonyl ausgewählt ist;
R⁵, R⁶ und R⁷ unabhängig aus Wasserstoff und C₁₋₃-Alkyl ausgewählt ist;
n für 0 oder 1 steht.

9. Verbindung nach Anspruch 7 oder 8, worin
X aus Wasserstoff, Fluor, Chlor, Brom, -CONH₂, Acetyl, C₁₋₃-Alkyl, C₁₋₃-Alkoxy, Cyclopropyl-C₁₋₃-alkyl und Cyclopropyl-C₁₋₃-alkoxy, worin das C₁₋₃-Alkyl, C₁₋₃-Alkoxy, Cyclopropyl-C₁₋₃-alkyl oder Cyclopropyl-C₁₋₃-alkoxy optional mit 1 bis 3 Fluoratomen substituiert ist, einem optional mit einem Methylrest substituiertem Heteroaryl, wobei das Heteroaryl aus Furyl, Pyrrolyl, Imidazolyl, Triazolyl, Isoxazolyl, Oxadiazolyl, Thiadiazolyl und Pyridinyl ausgewählt ist, ausgewählt ist;
Y aus Cyano, Wasserstoff und Fluor ausgewählt ist, wobei X und Y nicht Wasserstoff entsprechen;
R¹ für Wasserstoff steht.

10. Verbindung nach einem der Ansprüche 7 bis 9, worin
X aus Fluor, Methoxy, Ethoxy, Acetyl, -CONH₂, 2-Fluor-ethoxy, 2,2-Difluor-ethoxy, 2,2,2-Trifluor-ethoxy und Cyclopropyl-methoxy oder einem Heteroaryl ausgewählt ist, das aus 1-Imidazolyl, 5-Isoxazolyl, 3-Pyridinyl, 4-Pyridinyl und 5-Thiadiazolyl ausgewählt ist, wobei das Heteroaryl optional mit einem Methylrest substituiert ist;
Y für Fluor steht.

11. Pharmazeutische Zusammensetzung, die eine Verbindung nach einem der Ansprüche 1 bis 10 umfasst.

12. Verbindung nach einem der Ansprüche 1 bis 10 zur Verwendung in der Therapie.

13. Verbindung nach einem der Ansprüche 1 bis 10 zur Verwendung bei der Behandlung oder Verhinderung eines oder mehrerer Zustände bei einem Menschen, die aus entzündlicher Darmerkrankung, Colitis ulcerosa, gangrenöser Pyodermie, Morbus Crohn, Reizdarmsyndrom, spastischer Dystonie, chronischem Schmerz, akutem Schmerz, Bauchhöhlensprue, Pouchitis, Vasokonstriktion, Angst, Panikstörung, Depression, manisch-depressiver Erkrankung, Autismus, Schlafstörungen, Jetlag, amyotropher Lateralsklerose (ALS), kognitiver Dysfunktion, Bluthochdruck, Bulemie, Anorexie, Fettleibigkeit, Herzarrhytmien, Magensäure-Hypersekretion, Geschwüren, Pheochromocytom, progressiver supranukleärer Lähmung, chemischen Abhängigkeiten und Süchten, Abhängigkeiten von und Süchten nach Nikotin (oder Tabakprodukten), Alkohol, Benzodiazopinen, Barbituraten, Opioiden oder Kokain, Kopfschmerz, Migräne, Schlaganfall, traumatischer Gehirnverletzung (TBI), obsessiver-kompulsiver Störung (OCD), Psychose, Chorea-Huntington, Dyskinesia Tardive, Hyperkinesie, Dyslexie, Schizophrenie, Multi-Infarkt-Demenz, altersabhängiger Wahrnehmungsverringerung, Epilepsie, Petit-Mal-Epilepsie, seniler Demenz des Alzheimer-Typs (AD), Parkinson-Erkrankung (PD), hyperkinetischem Syndrom des Kindesalters (ADHD), Aufmerksamkeitsdefizitstörung (ADD), Eckbohm-Syndrom (RLS), schwachen kognitiven Beeinträchtigungen, Wahrnehmungserhöhung bei Schizophrenie, arzneimittelinduzierten extrapyramidalen Symptomen, Anpassungsstörungen, aufsässiger Trotzstörung, Angst bei ängstlichen Rauchern, kardiovaskulärem Risiko bei Schwangerschaft, verzögerter Ejakulation, Erbrechen, Diarrhöe, Nikotinkaugummisucht, Schlafverhinderung, Ischemie und Tourettte-Syndrom ausgewählt sind.

14. Verbindung nach einem der Ansprüche 1 bis 10 zur Verwendung bei der Behandlung oder Verhinderung von Schmerz bei einem Menschen.

## Revendications

1. Composé selon la formule I ou un sel de celui-ci, acceptable sur le plan pharmaceutique: 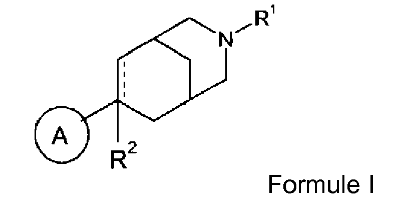 dans laquelle,
R¹ est un atome d'hydrogène, un groupe (alcoxy en C₁ à C₆)carbonyle, alkyle en C₁ à C₆, benzyloxycarbonyle, cyano(alkyle en C₁ à C₆), dihydro-3-pyridinylcarbonyle, hydroxy, hydroxyalkyle en C₁ à C₆, phenoxycarbonyle, -NR³R⁴, (NR³R⁴)(alkyle en C₁ à C₄), (NR³R⁴)carbonyl(alkyle en C₁ à C₄) ;
R² est présent sous forme de H ou OH ;
----- est une éventuelle double liaison, où lorsque ----- est une double liaison, R² est absent ;
A est un groupe aryle choisi parmi les groupes phényle, naphtyle, indényle, indanyle, azulényle et tétrahydronaphtyle,
où ledit groupe aryle est éventuellement substitué par un, deux, trois ou quatre substituants choisis indépendamment parmi les groupes alcényle en C₂ à C₆, alcoxy en C₁ à C₆, (alcoxy en C₁ à C₆)(alcoxy en C₁ à C₄), (alcoxy en C₁ à C₆)(alkyle en C₁ à C₄), (alcoxy en C₁ à C₆)carbonyle, (alcoxy en C₁ à C₆)carbonyl(alkyle en C₁ à C₄), alkyle en C₁ à C₆, (alkyle en C₁ à C₆)carbonyle, (alkyle en C₁ à C₆)carbonyloxy, alkylthio en C₁ à C₆, alcynyle en C₂ à C₆, carboxy, carboxy(alkyle en C₁ à C₆), cyano, cyano(alkyle en C₁ à C₆), cycloalkyle en C₃ à C₆, (cycloalkyle en C₃ à C₆)(alkyle en C₁ à C₄), (halogénocycloalkyle en C₃ à C₆)(alkyle en C₁ à C₄), (cycloalkyle en C₃ à C₆)(alcoxy en C₁ à C₄), halogéno(cycloalkyle en C₃ à C₆)(alcoxy en C₁ à C₄), formyle, formyl(alkyle en C₁ à C₆), halogénoalcoxy en C₁ à C₆, halogénoalkyle en C₁ à C₆, halogéno, hydroxy, hydroxyalkyle en C₁ à C₆, mercapto, mercaptoalkyle en C₁ à C₆, nitro, triphénylméthyle(trityle), un groupe hétéroaryle éventuellement substitué par un, deux ou trois substituants méthyle, où ledit groupe hétéroaryle est choisi parmi les groupes furyle, thiényle, pyrrolyle, pyrazolyle, imidazolyle, triazolyle, oxazolyle, isoxazolyle, thiazolyle, thiadiazolyle, isothiazolyle, oxadiazolyle, oxatriazolyle, pyridinyle, pyridazinyle, pyrimidinyle, pyrazinyle, triazinyle et tétrazolyle, -C(NH)NR³R⁴,-NR³R⁴, -(alkyle en C₁ à C₄)(NR³R⁴), -CO(NR³R⁴), -(alkyle en C₁ à C₄)CO(NR³R⁴), -S(O)₂NR³R⁴, -NR⁵S(O)₂R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂R⁵, phényle et phényl(CH₂)ₙO, où ledit groupe phényle ou phényl(CH₂)ₙO est éventuellement substitué par un, deux ou trois substituants choisis indépendamment parmi un atome de chlore, de fluor, un groupe cyano, alkyle en C₁ à C₃, alcoxy en C₁ à C₃, où ledit groupe alkyle en C₁ à C₃ ou alcoxy en C₁ à C₃ est éventuellement substitué par 1 à 3 atomes de fluor, ou bien où deux substituants sur des atomes de carbone adjacents dudit groupe aryle représentent ensemble éventuellement -OCH₂CH₂-, -OCH₂CH(OH), -OCH₂CH₂O- ou -OCH₂O-;
R³ et R⁴ sont choisis indépendamment parmi un atome d'hydrogène, un groupe alkyle en C₁ à C₄, (alkyle en C₁ à C₄)carbonyle ;
R⁵, R⁶ et R⁷ sont choisis indépendamment parmi un atome d'hydrogène, un groupe alkyle en C₁ à C₄, phényle, et phényl(alkyle en C₁ à C₄) ;
n vaut 0, 1 ou 2.

2. Composé selon la revendication 1, dans lequel A est un groupe phényle éventuellement substitué par un, deux, trois ou quatre substituants choisis indépendamment parmi les groupes alcényle en C₂ à C₆, alcoxy en C₁ à C₆, (alcoxy en C₁ à C₆)(alcoxy en C₁ à C₄), (alcoxy en C₁ à C₆)(alkyle en C₁ à C₄), (alcoxy en C₁ à C₆)carbonyle, (alcoxy en C₁ à C₆)carbonyl(alkyle en C₁ à C₄), alkyle en C₁ à C₆, (alkyle en C₁ à C₆)carbonyle, (alkyle en C₁ à C₆)carbonyloxy, alkylthio en C₁ à C₆, alcynyle en C₂ à C₆, carboxy, carboxy(alkyle en C₁ à C₆), cyano, cyano(alkyle en C₁ à C₆), cycloalkyle en C₃ à C₆, (cycloalkyle en C₃ à C₆)(alkyle en C₁ à C₄), formyle, formyl(alkyle en C₁ à C₆), halogénoalcoxy en C₁ à C₆, halogénoalkyle en C₁ à C₆, halogéno, hydroxy, hydroxyalkyle en C₁ à C₆, mercapto, mercaptoalkyle en C₁ à C₆, nitro, triphénylméthyle(trityle), furyle, thiényle, pyrrolyle, pyrazolyle, imidazolyle, triazolyle, oxazolyle, isoxazolyle, thiazolyle, thiadiazolyle, isothiazolyle, oxadiazolyle, oxatriazolyle, pyridinyle, pyridazinyle, pyrimidinyle, pyrazinyle, triazinyle, tétrazolyle, -C(NH)NR³R⁴, -NR³R⁴, -(alkyle en C₁ à C₄)(NR³R⁴), -CO(NR³R⁴), -(alkyle en C₁ à C₄)CO(NR³R⁴), -S(O)₂NR³R⁴, -NR⁵S(O)₂R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂R⁵, phényle et phényl(CH₂)ₙO, où ledit groupe phényle ou phényl(CH₂)ₙO est éventuellement substitué par un, deux ou trois substituants choisis indépendamment parmi un atome de chlore, de fluor, un groupe cyano, ou bien où deux substituants sur des atomes de carbone adjacents dudit groupe aryle représentent ensemble éventuellement -OCH₂CH₂-, -OCH₂CH(OH), -OCH₂CH₂O- ou -OCH₂O-.

3. Composé selon la revendication 1 ou la revendication 2, dans lequel
R¹ est H, un groupe (alcoxy en C₁ à C₃)carbonyle, alkyle en C₁ à C₃, cyano(alkyle en C₁ à C₃), hydroxy, hydroxyalkyle en C₁ à C₃;
A est un groupe phényle éventuellement substitué par un, deux ou trois substituants choisis indépendamment parmi un groupe amide, alcényle en C₂ à C₄, alcoxy en C₁ à C₃, (alcoxy en C₁ à C₃)(alkyle en C₁ à C₃), (alcoxy en C₁ à C₃)carbonyle, alkyle en C₁ à C₃, (alkyle en C₁ à C₃)carbonyle, (alkyle en C₁ à C₃)carbonyloxy, alkylthio en C₁ à C₃, alcynyle en C₂ à C₄, carboxy, carboxy(alkyle en C₁ à C₃), cyano, cyano(alkyle en C₁ à C₃), formyle, formyl(alkyle en C₁ à C₃), halogénoalcoxy en C₁ à C₃, halogénoalkyle en C₁ à C₃, , halogéno, hydroxy, hydroxyalkyle en C₁ à C₃, mercapto, mercaptoalkyle en C₁ à C₃, nitro, furyle, thiényle, pyrrolyle, pyrazolyle, imidazolyle, triazolyle, oxazolyle, isoxazolyle, thiazolyle, thiadiazolyle, isothiazolyle, oxadiazolyle, oxatriazolyle, pyridinyle, pyridazinyle, pyrimidinyle, pyrazinyle, triazinyle, tétrazolyle, phényle et phényl(CH₂)ₙO, où ledit groupe phényle ou phényl(CH₂)ₙO est éventuellement substitué par un, deux ou trois substituants choisis indépendamment parmi un atome de chlore, de fluor, un groupe cyano, où n vaut 0, 1 ou 2.

4. Composé selon la revendication 3, dans lequel R¹ est H, un groupe alkyle en C₁ à C₃, ou hydroxy

5. Composé selon l'une quelconque des revendications 1 à 4, dans lequel
R¹ est H ou un groupe méthyle ;
A est un groupe phényle éventuellement substitué par un, deux ou trois substituants choisis indépendamment parmi un groupe cyano, un atome de fluor, de chlore, de brome, un groupe méthyle, éthyle, isopropyle, méthoxy, éthoxy, isopropyloxy, benzyloxy, trifluorométhyle, difluorométhoxy, trifluorométhoxy, phénoxy, 4-chlorophénoxy, 4-cyanophénoxy et 4-fluorophénoxy.

6. Composé selon la revendication 5, dans lequel A est un groupe phényle choisi parmi un groupe 2-fluorophényle, 3-fluorophényle, 3,5-difluorophényle, 3,4-difluorophényle, 3,4,5-trifluorophényle, 3-cyanophényle et 3-méthoxyphényle.

7. Composé selon la revendication 1, dans lequel ledit composé possède la formule III : 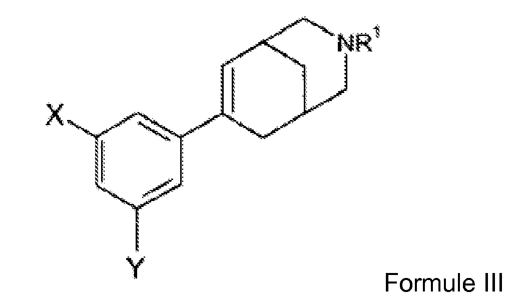 dans laquelle X est choisi parmi
un groupe alkyle en C₁ à C₆, alcényle en C₂ à C₆, alcoxy en C₁ à C₆, (alcoxy en C₁ à C₆)(alcoxy en C₁ à C₄), (alcoxy en C₁ à C₆)(alkyle en C₁ à C₄), (alcoxy en C₁ à C₆)carbonyle, (alcoxy en C₁ à C₆)carbonyl(alkyle en C₁ à C₄), (alkyle en C₁ à C₆)carbonyle, (alkyle en C₁ à C₆)carbonyloxy, alkylthio en C₁ à C₆, alcynyle en C₂ à C₆, carboxy, carboxy(alkyle en C₁ à C₆), cyano, cyano(alkyle en C₁ à C₆), cycloalkyle en C₃ à C₆, (cycloalkyle en C₃ à C₆)(alkyle en C₁ à C₄), (cycloalkyle en C₃ à C₆)(alcoxy en C₁ à C₄), où ledit groupe (cycloalkyle en C₃ à C₆)(alkyle en C₁ à C₄) ou (cycloalkyle en C₃ à C₆)(alcoxy en C₁ à C₄) est éventuellement substitué par 1 à 3 atomes d'halogène ; formyl(alkyle en C₁ à C₆), halogénoalcoxy en C₁ à C₆, halogénoalkyle en C₁ à C₆, un atome d'halogène, un atome d'hydrogène, un groupe hydroxy, hydroxyalkyle en C₁ à C₆, carboxy, mercapto, mercaptoalkyle en C₁ à C₆, nitro, triphénylméthyle(trityle), C(NH)NR³R⁴, -NR³R⁴, -(alkyle en C₁ à C₄)(NR³R⁴), -CO(NR³R⁴), -(alkyle en C₁ à C₄)CO(NR³R⁴), -S(O)₂NR³R⁴, -NR⁵S(O)₂R⁶, -C(NR⁵)NR⁶R⁷,-CH₂C(NR⁵)NR⁶R⁷, -C(NOR⁵)R⁶, -C(NCN)R⁵, -C(NNR⁵R⁶)R⁷, -S(O)₂OR⁵,-S(O)₂R⁵, un groupe hétéroaryle éventuellement substitué par un deux ou trois substituants méthyle, où ledit groupe hétéroaryle est choisi parmi les groupes furyle, thiényle, pyrrolyle, pyrazolyle, imidazolyle, triazolyle, oxazolyle, isoxazolyle, thiazolyle, thiadiazolyle, isothiazolyle, oxadiazolyle, oxatriazolyle, pyridinyle, pyridazinyle, pyrimidinyle, pyrazinyle, triazinyle et tétrazolyle, phényle et phényl(CH₂)ₙO, où ledit groupe phényle ou phényl(CH₂)ₙO est éventuellement substitué par un, deux ou trois substituants choisis indépendamment parmi un atome de chlore, de fluor, un groupe cyano, un groupe alkyle en C₁ à C₃ et alcoxy en C₁ à C₃ où ledit groupe alkyle en C₁ à C₃ ou alcoxy en C₁ à C₃ est éventuellement substitué par 1 à 3 atomes de fluor ;
Y est choisi parmi un groupe cyano, un atome d'hydrogène, de fluor, de chlore, de brome ;
R¹ est choisi parmi un atome d'hydrogène et un groupe alkyle en C₁ à C₄;
R³ et R⁴ sont choisis indépendamment parmi un atome d'hydrogène, un groupe alkyle en C₁ à C₄ et (alkyle en C₁ à C₄)carbonyle ;
R⁵,R⁶ et R⁷ sont choisis indépendamment parmi un atome d'hydrogène, un groupe alkyle en C₁ à C₄, phényle et phényl(alkyle en C₁ à C₄).

8. Composé selon la revendication 7, dans lequel X est choisi parmi un groupe alkyle en C₁ à C₃, alcényle en C₂ à C₄, alcoxy en C₁ à C₃, (alcoxy en C₁ à C₃)(alcoxy en C₁ à C₃), (alcoxy en C₁ à C₃)(alkyle en C₁ à C₃), (alcoxy en C₁ à C₃)carbonyle, (alcoxy en C₁ à C₃)carbonyl(alkyle en C₁ à C₃), (alkyle en C₁ à C₃)carbonyle, (alkyle en C₁ à C₃)carbonyloxy, alkylthio en C₁ à C₃, alcynyle en C₂ à C₄, carboxy, cyano, cyano(alkyle en C₁ à C₃), cyclopropyle, (cyclopropyl)(alkyle en C₁ à C₃), (cyclopropyl)(alcoxy en C₁ à C₃), où ledit groupe alkyle en C₁ à C₃, alcoxy en C₁ à C₃, (cyclopropyl)(alkyle en C₁ à C₃), (cyclopropyl)(alcoxy en C₁ à C₃) est éventuellement substitué par 1 à 3 atomes d'halogène choisis indépendamment parmi un atome de fluor et un atome de chlore; formyl(alkyle en C₁ à C₃), un atome d'halogène, un atome d'hydrogène, un groupe hydroxy, hydroxyalkyle en C₁ à C₃, mercapto, mercaptoalkyle en C₁ à C₃, nitro, C(NH)NR³R⁴, -NR³R⁴, - (alkyle en C₁ à C₃)(NR³R⁴), -CO(NR³R⁴), -(alkyle en C₁ à C₃)CO(NR³R⁴), -S(O)₂NR³R⁴, -NR⁵S(O)₂R⁶, -C(NR⁵)NR⁶R⁷, -CH₂C(NR⁵)NR⁶R⁷, -C(NOR⁵)R⁶, -C(NCN)R⁵, -C(NNR⁵R⁶)R⁷, -S(O)₂OR⁵, -S(O)₂R⁵, un groupe hétéroaryle éventuellement substitué par un deux ou trois substituants méthyle, où ledit groupe hétéroaryle est choisi parmi les groupes furyle, thiényle, pyrrolyle, pyrazolyle, imidazolyle, triazolyle, oxazolyle, isoxazolyle, thiazolyle, thiadiazolyle, isothiazolyle, oxadiazolyle, pyridinyle, pyridazinyle, pyrimidinyle et pyrazinyle, phényle et phényl(CH₂)ₙO, où ledit groupe phényle ou phényl(CH₂)ₙO est éventuellement substitué par un, deux ou trois substituants choisis indépendamment parmi un atome de fluor, un groupe cyano, méthyle, éthyle, méthoxy et éthoxy, où ledit groupe méthyle, éthyle, méthoxy et éthoxy est éventuellement substitué par 1 à 3 atomes de fluor ;
Y est choisi parmi un groupe cyano, un atome d'hydrogène, de fluor et de chlore ;
R¹ est choisi parmi un groupe méthyle et un atome d'hydrogène;
R³ et R⁴ sont choisis indépendamment parmi un atome d'hydrogène, un groupe alkyle en C₁ à C₃ et (alkyle en C₁ à C₃)carbonyle ;
R⁵,R⁶ et R⁷ sont choisis indépendamment parmi un atome d'hydrogène, un groupe alkyle en C₁ à C₃ ;
n vaut 0 ou 1.

9. Composé selon la revendication 7 ou la revendication 8, dans lequel
X est choisi parmi un atome d'hydrogène, de fluor, de chlore, de brome, un groupe -CONH₂, acétyle, alkyle en C₁ à C₃, alcoxy en C₁ à C₃, cyclopropyl(alkyle en C₁ à C₃) et cyclopropyl(alcoxy en C₁ à C₃) où ledit groupe alkyle en C₁ à C₃, alcoxy en C₁ à C₃, cyclopropyl(alkyle en C₁ à C₃) ou cyclopropyl(alcoxy en C₁ à C₃) est éventuellement substitué par 1 à 3 atomes de fluor, un groupe hétéroaryle éventuellement substitué par un groupe méthyle, où ledit groupe hétéroaryle est choisi parmi les groupes furyle, pyrrolyle, imidazolyle, triazolyle, isoxazolyle, oxadiazolyle, thiadiazolyle et pyridinyle ;
Y est choisi parmi un groupe cyano, un atome d'hydrogène et un atome de fluor, où X et Y ne sont pas égaux à un atome d'hydrogène ;
R¹ est un atome d'hydrogène.

10. Composé selon l'une quelconque des revendications 7 à 9, dans lequel
X est choisi parmi un atome de fluor, un groupe méthoxy, éthoxy, acétyle, -CONH₂, 2-fluoro-éthoxy, 2,2-difluoro-éthoxy, 2,2,2-trifluoro-éthoxy et cyclopropyl-méthoxy ou un groupe hétéroaryle choisi parmi un groupe 1-imidazolyle, 5-isoxazolyle, 3-pyridinyle, 4-pyridinyle et 5-thiadiazolyle, où ledit groupe hétéroaryle est éventuellement substitué par un groupe méthyle ;
Y est un atome de fluor.

11. Composition pharmaceutique comprenant un composé selon l'une quelconque des revendications 1 à 10.

12. Composé selon l'une quelconque des revendications 1 à 10 pour une utilisation en thérapie.

13. Composé selon l'une quelconque des revendications 1 à 10 pour une utilisation dans le traitement ou la prévention d'un ou plusieurs états chez un humain choisis parmi l'affection abdominale inflammatoire, la colite ulcéreuse, le pyoderme gangréneux, la maladie de Crohn, la colopathie fonctionnelle, la dystonie spastique, la douleur chronique, la douleur aiguë, la sprue coeliaque, la pochite, la vasoconstriction, l'anxiété, le trouble panique, la dépression, le trouble bipolaire, l'autisme, les troubles du sommeil, les troubles dus au décalage horaire, la sclérose latérale amyotrophique (SLA), le dysfonctionnement cognitif, l'hypertension, la boulimie, l'anorexie, l'obésité, les arythmies cardiaques, l'hypersécrétion d'acide gastrique, les ulcères, le phéochromocytome, la paralysie supranucléaire progressive, les dépendances et addictions chimiques, les dépendances ou addictions à la nicotine (ou aux produits du tabac), à l'alcool, aux benzodiazopines, aux barbituriques, aux opioïdes ou à la cocaïne, le mal de tête, la migraine, l'attaque, les lésions cérébrales traumatiques (TBI), le trouble obsessionnel-compulsif (TOC), la psychose, la chorée de Huntington, la dyskinésie tardive, l'hyperkinésie, la dyslexie, la schizophrénie, la démence multi-infarctus, le déclin cognitif lié à l'âge, l'épilepsie, l'épilepsie de petit mal absence, la démence sénile du type Alzheimer (MA), la maladie de Parkinson (MP), le trouble de déficit de l'attention/hyperactivité (TDA/H), le trouble de déficit de l'attention (TDA), le syndrome des jambes sans repos (RLS), le déclin cognitif modéré, l'amélioration cognitive dans la schizophrénie, les symptômes extrapyramidaux induits par les médicaments, le trouble du comportement, le trouble oppositionnel avec provocation, l'anxiété chez les fumeurs anxieux, le risque cardiovasculaire pendant la grossesse, l'éjaculation retardée, le vomissement, la diarrhée, l'addiction à la gomme nicotinique, l'empêchement du sommeil, l'ischémie et le syndrome de Tourette.

14. Composé selon l'une quelconque des revendications 1 à 10 pour une utilisation dans le traitement ou la prévention de la douleur chez l'humain.
